(19) Europäisches Patentamt

European Patent Office

Office européen des brevets

(11) **EP 1 096 921 B1**

(12) **EUROPEAN PATENT SPECIFICATION**

(45) Date of publication and mention
of the grant of the patent:
**16.04.2003 Bulletin 2003/16**

(21) Application number: **99934114.2**

(22) Date of filing: **16.07.1999**

(51) Int Cl.⁷: **A61K 9/127**, A61K 48/00

(86) International application number:
**PCT/US99/16166**

(87) International publication number:
**WO 00/003683 (27.01.2000 Gazette 2000/04)**

(54) **LIPOSOMAL ENCAPSULATED NUCLEIC ACID-COMPLEXES**

IN LIPOSOMEN VERKAPSELTE NUKLEINSÄUREKOMPLEXE

COMPLEXES D'ACIDES NUCLEIQUES ENCAPSULES DANS DES LIPOSOMES

(84) Designated Contracting States:
**AT BE CH CY DE DK ES FI FR GB GR IE IT LI LU
MC NL PT SE**

(30) Priority: **20.07.1998 US 93481 P**

(43) Date of publication of application:
**09.05.2001 Bulletin 2001/19**

(73) Proprietor: **Protiva Biotherapeutics Inc.
Burnaby, British Columbia V5G 4Y1 (CA)**

(72) Inventors:
• **BOEY, Anthony
Port Coquitlam, British Columbia V3C 6J8 (CA)**
• **CHEN, Tao
Richmond, British Columbia V64 1Z3 (CA)**
• **LENG, Esther
Surrey, British Columbia V3W 3L6 (CA)**

• **TARD, Paul, G.
Surrey, British Columbia V3S 7M6 (CA)**
• **WHEELER, Jeffrey, J.
Surrey, British Columbia V34 8G9 (CA)**
• **SCHERRER, Peter
Vancouver, British Columbia V6H 2T5 (CA)**
• **GRAHAM, Roger
Vancouver, British Columbia V6K 1Z1 (CA)**

(74) Representative: **West, Alan Harry et al
R.G.C. Jenkins & Co.
26 Caxton Street
London SW1H 0RJ (GB)**

(56) References cited:
**WO-A-95/34647         WO-A-98/20857
WO-A-98/34648         WO-A-99/58694**

**Description**

This invention relates to liposomes that are useful for introducing nucleic acids into cells. More particularly, the liposomes of the present invention entrap a condensing agent-nucleic acid complex and, thus, they are useful as nucleic acid-transfer delivery vehicles in clinical use.

The introduction of foreign genes and other molecules into cells is of great interest to molecular biologists. One reason to introduce genetic material into cells is to express an encoded protein. Gene transfer involves delivering nucleic acids to target cells and then transferring the nucleic acid across the cell membrane in a form that can function in a therapeutic manner. Of the many methods used to facilitate entry of DNA into eukaryotic cells, liposomes are among the most efficacious and have found extensive use as DNA carriers in transfection experiments. Cationic lipids are known to bind to polynucleotides and to facilitate their intracellular delivery into mammalian cells. Nucleic acid is negatively charged and when combined with a positively charged lipid, forms a complex that is suitable for formulation and cellular delivery. The use of cationic lipid carriers for transfection is well established.

Other gene transfer methods under study include viral vectors. Although viral vectors have the inherent ability to transport nucleic acids across cell membranes and, in some instances, integrate exogenous DNA into chromosomes, they can carry only limited amounts of DNA and pose several risks. One such risk involves the random integration of viral genetic sequences into patient chromosomes, potentially damaging the genome and possibly inducing a malignant transformation. Another risk is that the viral vector may revert to a pathogenic genotype either through mutation or genetic exchange with a wild type virus.

Limitations associated with viral gene delivery systems have spawned the development of nonviral gene transfer vectors. These nonviral systems generally consist of plasmid DNA complexed to a cationic agent, such as a lipid or polymer, to condense the nucleic acid and to facilitate its cellular uptake into the cell membrane. One of the obstacles to gene expression is the degradation of the DNA in route to the nucleus within the cytoplasm. In this respect, polycations have been used extensively to overcome this obstacle and improve gene expression. These cationic polymers include antibiotics, such as gramicidin S, dendrimers or cascade polymers or cationically modified albumin. In addition, spermidine has been shown to condense DNA and improve transfection of cell cultures. These condensing agents protect the DNA from degradation by endonucleases and restriction enzymes. The positive charge on these polymers is also expected to boost the transfection capability of the complexes.

Other polycationic polymers that are useful as condensing agents because of their affinity to electrostatically bind nucleic acids include polylysine, polyarginine and polyornithine. The polycation polyethylenimine (PEI), which is a highly-branched polymer, has been shown to be a highly efficient gene delivery agent. In this regard, PEI condenses nucleic acid into a highly compact form and offers good protection from various nucleases. It has been reported that the gene transfer with these complexes was boosted up to a 1000-fold, under certain conditions. Clearly, polycations like PEI have a clear advantage over the lipid/nucleic acid complexes in this respect.

However, one significant drawback of polycationic-nucleic acid complexes, such as a PEI-nucleic acid complex, is the toxicity associated with *in vivo* gene delivery via the use of such complexes. When PEI is condensed with nucleic acid at higher ratios, the complexes become toxic. At lower ratios (∼2), transfection is reduced significantly. If these highly transfecting particles are to be used *in vivo* for transfection, the toxicity must be reduced to tolerable levels.

As such, there exists a need to design condensing agent-nucleic acid complexes that are effective for facilitating intracellular delivery of genetic material, but that will reduce the associated cellular toxicity.

WO 95/34647A describes compositions and methods for enhancing delivery of nucleic acids to cells, on the basis of complexes of the new nucleic acids bound to nuclear localisation peptides, for example histones. Also, WO 98/20857A describes lipid-nucleic acid complex formulations for *in vivo* delivery, such formulations being prepared by combining a preformed liposome with a condensed nucleic acid.

The present invention provides lipid-nucleic acid complexes which, as described in detail below, differ from and exhibit clear advantages over the complexes described above.

In one aspect, the present invention provides a liposome having (a) a lipid; and (b) an encapsulated condensing agent-nucleic acid complex. In certain preferred aspects, the liposomes of the present invention further comprise (c) a bilayer stabilizing component. The bilayer stabilizing component can be reversibly associated with the liposome. Such liposomes are extremely advantageous because they offer good protection to the nucleic acid from various nucleases that tend to degrade nucleic acid that is not protected by encapsulation. Moreover, in many instances, gene transfer with these complexes is increased up to a 1000-fold. In addition, using the encapsulating formulations of the present invention the toxicity of the condensing agents are reduced to tolerable levels.

Condensing agents suitable for use in the present invention include, but are not limited to, polycationic polymers, such as polyethylenimine, polylysine, polyarginine and polyornithine. Other condensing agents that have an affinity for nucleic acid and that are suitable for use in the present invention include, but are not limited to, natural DNA-binding proteins of a polycationic nature, such as histones and protamines or analogues or fragments thereof. Other condensing agents suitable for use in the present invention include spermidine, spermine, polycations having two or more different positively charged amino acids or basic proteins.

Although numerous lipids can be used, the lipids used in the liposomes of the present invention are preferably non-cationic lipids. Such non-cationic lipids include, but are not limited to, ceramides, phosphatidylethanolamines, phosphatidylserines and mixtures thereof. In a presently preferred embodiment, the non-cationic lipids used are ceramides, dioleoylphosphatidylethanolamine, dioleoylphosphatidylserine and mixtures thereof.

In another aspect, the present invention relates to a method for encapsulating a condensing agent-nucleic acid complex in a liposome, the method comprising: adding a condensing agent solution into a nucleic acid solution to form a condensing agent-nucleic acid complex; and adding said condensing agent-nucleic acid complex to a lipid suspension to form an encapsulated condensing agent-nucleic acid complex. In a preferred embodiment, the method comprises:

(a) admixing a first condensing agent solution into a nucleic acid solution to form precondensed nucleic acid;
(b) adding the precondensed nucleic acid into a second condensing agent solution to form a condensing agent-nucleic acid complex;
(c) dialyzing the condensing agent-nucleic acid complex to form a concentrated condensing agent-nucleic acid complex;
(d) adding the concentrated condensing agent-nucleic acid complex to a lipid suspension containing detergent; and
(e) removing the detergent from the lipid suspension to form an encapsulated condensing agent-nucleic acid complex in the liposome.

In this method, the first and second condensing agents can be the same or different.

In yet another aspect, the present invention relates to a method of transfecting a cell with a nucleic acid in vitro, the method comprises contacting the cell with a liposome having (a) a lipid; and (b) an encapsulated condensing agent-nucleic acid complex. In certain preferred embodiments, the liposomes of this method further comprise (c) a bilayer stabilizing component. The bilayer stabilizing component can be reversibly associated with the liposome.

In still yet another aspect, this invention relates to the treatment of a disease involving the transfection of a cell with nucleic acid and the introduction into cells of antisense nucleotides, as well as the stable transfection of a cell with DNA engineered to become incorporated into the genome of the living cell.

Other features, objects and advantages of the invention and its preferred emb diments will become apparent from the detailed description which follows, and which makes reference to the accompanying drawings, in which:

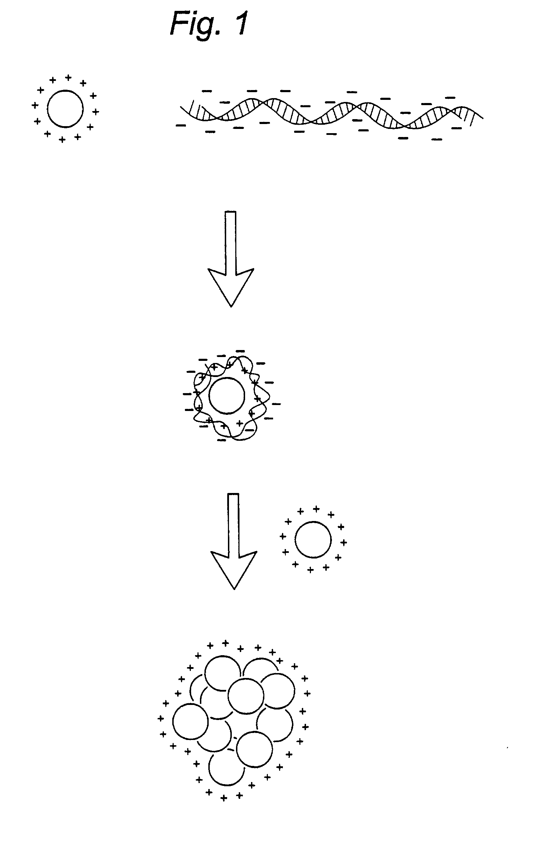

**Figure 1** illustrates the construction of uniformed small size polyethylenimine-nucleic acid complex;
**Figure 2** illustrates an effect of dextran sulfate on PEI-DNA complexes;
**Figure 3** illustrates the titration of dextran sulfate to determine the minimal amount of dextran sulfate required to completely expose DNA to picogreen;
**Figure 4** illustrates a standard curve for quantifying DNA At each data point, a standard amount of dextran sulfate is added, which is the same amount added to the random test samples of complexes;
**Figure 5** illustrates that the relaxation or dissociation of the complexes is not an instantaneous event;
**Figure 6** illustrates the extent of encapsulation when dextran sulfate is used in the assay to dissociate the nucleic acid from the PEI;
**Figure 7** illustrates a titration of DOPS to optimize encapsulation efficiency;

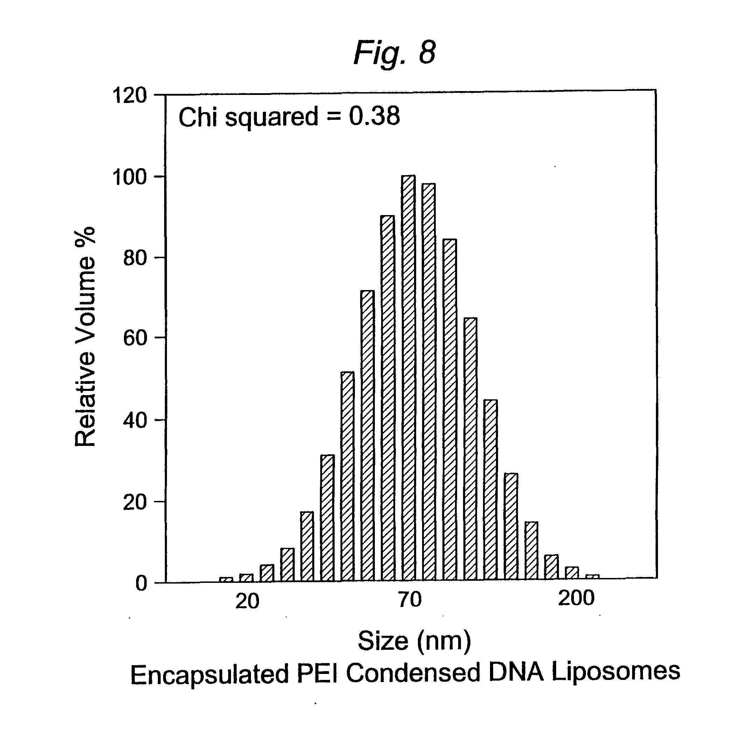

**Figure 8** illustrates a Guassian size distribution of a sample of lipid encapsulated PEI/DNA containing 8 mol% DOPS. The liposomes are typically around 75 to about 80 nm in diameter;
**Figure 9** illustrates transfection of Cos-7 cells with encapsulated PEI condensed DNA liposomes-dose response and time course;
**Figure 10** illustrates transfection of various cell lines with encapsulated PEI condensed DNA Liposomes. LS-180 is derived from a 58 year old female patient with the Duke's type adenocarcinoma of the colon; SK-OV-3 is a human ovarian adenocarcinoma tumor taken from a 64 year old; U87 is human glioblastoma; COS-7 is kidney, fibroblast-like cell line established from CV-1 simian cells which were transformed by an origin-defective mutant of SV40; Lewis Lung is human lung carcinoma; and B16 is mouse melanoma;
**Figure 11** illustrates an *in vitro* toxicity of encapsulated PEI condensed DNA liposomes in Cos-7 cell line;
**Figure 12** illustrates concentration dependence of the condensing agent-nucleic acid complex on cell death;

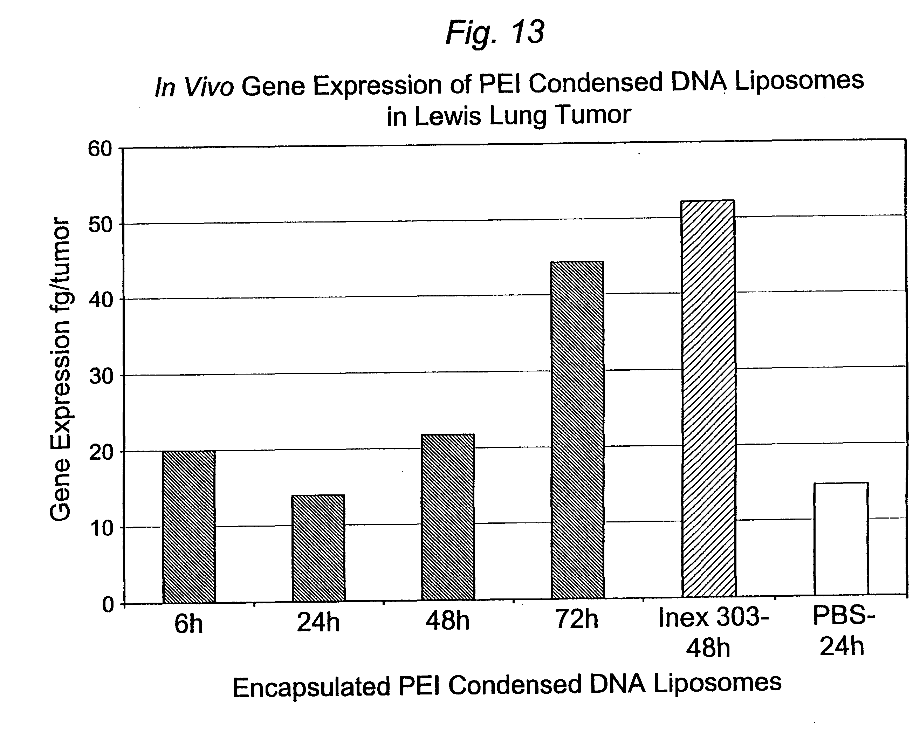

**Figure 13** illustrates *in vivo* gene expression of PEI condensed DNA liposomes in Lewis Lung tumor;

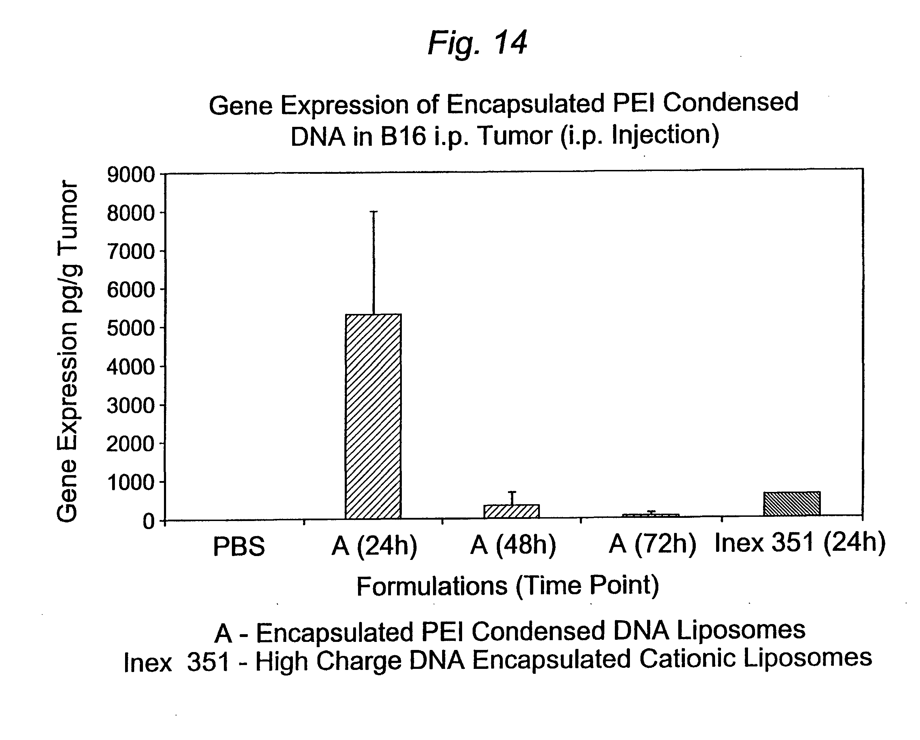

**Figure 14** illustrates gene expression of encapsulated PEI condensed DNA in B16 i.p. tumor; and

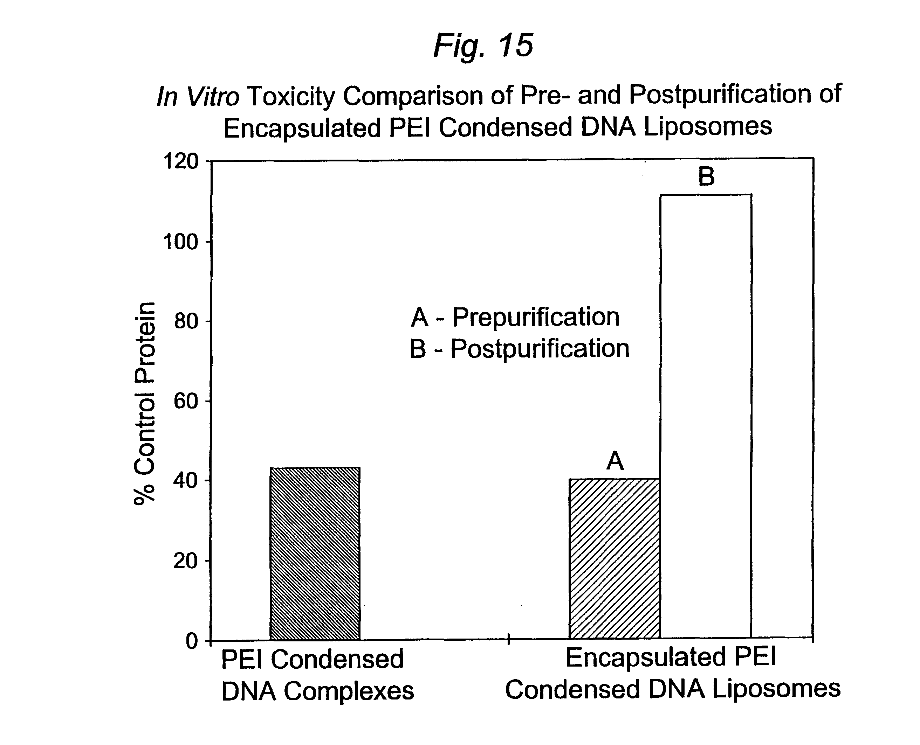

**Figure 15** illustrates *in vitro* toxicity of pre and post purification of encapsulated PEI condensed DNA liposomes.

## A. Glossary

The term "lipid" refers to any suitable material resulting in a bilayer such that a hydrophobic portion of the lipid material orients toward the bilayer while a hydrophilic portion orients toward the aqueous phase. Amphipathic lipids have a hydrophilic portion and a hydrophobic portion. Hydrophilic characteristics derive from the presence of phosphato, carboxylic, sulfato, amino, sulfhydryl, nitro, and other like groups. Hydrophobicity could be conferred by the inclusion of groups that include, but are not limited to, long chain saturated and unsaturated aliphatic hydrocarbon groups and such groups substituted by one or more aromatic, cycloaliphatic or heterocyclic group(s). Amphipathic compounds include, but are not limited to, phosphoglycerides and sphingolipids, representative examples of which include phosphatidylcholine, phosphatidylethanolamine, phosphatidylserine, phosphatidylinositol, phosphatidic acid, phosphatidylcholine, lysophosphatidylcholine, lysophosphatidylethanolamine, dipalmitoylphosphatidylcholine, dioleoylphosphatidylcholine, distearoylphosphatidylcholine or dilinoleoylphosphatidylcholine. Other compounds lacking in phosphorus, such as sphingolipid and glycosphingolipid families are also within the group designated as lipid. Additionally, the amphipathic lipids described above may be mixed with other lipids including triglycerides and sterols.

The term "neutral lipid" refers to any of a number of lipid species which exist either in an uncharged or neutral zwitterionic form at physiological pH. Such lipids include, for example, diacylphosphatidylcholine, diacylphosphatidylethanolamine, ceramide, sphingomyelin, cephalin, and cerebrosides.

The term "non-cationic lipid" refers to any neutral lipid as described above as well as anionic lipids. Preferred non-cationic lipids include phosphatidylethanolamines, phosphatidylserines and ceramides. Examples of preferred anionic lipids include cardiolipin, diacylphosphatidylserine, diacylphosphatidic acid, N-succinyl-phosphatydylethanolamine (N-succinyl-PE), phosphatidic acid, phosphatidylinositol, phosphatidylglycerol and phosphatidyl ethylene glycol.

The term "cationic lipid" refers to any of a number of lipid species which carry a net positive charge at physiological pH. Such lipids include, but are not limited to, DODAC, DOTMA, DDAB, DOTAP, DC-Chol and DMRIE. Additionally, there are a number of commercial preparations of cationic lipids. These include, for example, LIPOFECTIN® (commercially available cationic liposomes comprising DOTMA and DOPE, from GIBCO/BRL, Grand Island, New York, USA); LIPOFECTAMINE® (commercially available cationic liposomes comprising DOSPA and DOPE, from GIBCO/BRL); and TRANSFECTAM® (commercially available cationic lipids comprising DOGS in ethanol from Promega Corp., Madison, Wisconsin, USA).

The term "bilayer stabilizing component" as used herein refers to compounds (*e.g.*, lipids, polymers, *etc.*) that allow lipids adopting a non-lamellar phase under physiological conditions to be stabilized in a bilayer structure. The bilayer stabilizing components are either bilayer forming themselves, or are of a complementary dynamic shape. The non-bilayer forming lipid is stabilized in the bilayer structure when it is associated with, *i.e.*, in the presence of, the bilayer stabilizing component. In certain embodiments the bilayer stabilizing component is capable of transferring out of the liposome, or of being chemically modified by endogenous systems such that, with time, it loses its ability to stabilize the lipid in a bilayer structure. When liposomal stability is lost, destabilized or decreased, fusion can occur. Fusion can result in the release of liposome payload into the target cell. Thus, in certain embodiments, the bilayer stabilizing component is, "reversibly associated" with the lipid and when it is associated with the lipid, the lipid is constrained to adopt the bilayer structure under conditions where it would otherwise adopt a non-lamellar phase. As such, the bilayer stabilizing components of the present invention is capable of stabilizing the lipid in a bilayer structure, yet is capable of exchanging out of the liposome, or of being chemically modified by endogenous systems so that, with time, they lose their ability to stabilize the lipid in a bilayer structure, thereby allowing the liposome to become fusogenic or release its payload.

In certain other embodiments, the bilayer stabilizing component does not transfer out of the liposome. In these embodiments, the liposome is non-fusogenic and the bilayer stabilizing component is not, "reversibly associated" with the lipid.

The term "transfection" as used herein, refers to the introduction of polyanionic materials, particularly nucleic acids, into cells. The term "lipofection" refers to the introduction of such materials in association with lipids. The polyanionic materials can be in the form of DNA or RNA which is linked to expression vectors to facilitate gene expression after entry into the cell. Thus, the polyanionic material used in the present invention is meant to include DNA having coding sequences for structural proteins, receptors and hormones, as well as transcriptional and translational regulatory elements (*i.e.*, promoters, enhancers, terminators and signal sequences) and vector sequences. Methods of incorporating particular nucleic acids into expression vectors are well known to those of skill in the art, but are described in detail in, for example, Sambrook *et al., Molecular Cloning: A Laboratory Manual* (2nd ed.), Vols. 1-3, Cold Spring Harbor Laboratory, (1989) or *Current Protocols in Molecular Biology, F.* Ausubel *et al.,* ed. Greene Publishing and Wiley-Interscience, New York (1987).

"Expression vectors," "cloning vectors," or "vectors" are often plasmids or other nucleic acid molecules that are able to replicate in a chosen host cell. Expression vectors may replicate autonomously, or they may replicate by being inserted into the genome of the host cell, by methods well known in the art. Vectors that replicate autonomously will have an origin of replication or autonomous replicating sequence (ARS) that is functional in the chosen host cell (s). Often, it is desirable for a vector to be usable in more than one host cell, *e.g.,* in *E. coli* for cloning and construction, and in a mammalian cell for expression.

The term "encapsulation" as used herein when discussing amount of encapsulation, refers to the amount of condensing agent-nucleic acid complex that is unavailable to picogreen binding in a picogreen/dextran binding assay or that is nuclease resistant in a nuclease assay.

**B. General**

It has now been discovered that lipid encapsulation of a condensing agent-nucleic acid complex offers greater protection against enzymatic digestion and gives consistently higher gene expression than unencapsulated condensing agent-nucleic acid complexes. As such, in one aspect, the present invention relates to a liposome comprising (a) a lipid; and (b) an encapsulated condensing agent-nucleic acid complex. In certain preferred embodiments, the liposome further comprises (c) a bilayer stabilizing component. The bilayer stabilizing component can be reversibly associated with the liposome.

The condensing agents used in the liposomes of the present invention can be any compound that has the ability to complex and compact nucleic acids. The complex generally comprises at least one negatively charged nucleic acid and at least one positively charged polymer, the association between the nucleic acid and the cationic polymer being electrostatic in nature.

As such, the condensing agents suitable for use in the present invention include, but are not limited to, polycationic polymers, such as polyethylenimine, polylysine, polyarginine and polyornithine. Other condensing agents that have an affinity for nucleic acid and that are suitable for use in the present invention include, but are not limited to, natural DNA-binding proteins of a polycationic nature, such as histones and protamines or analogues or fragments thereof Other condensing agents suitable for use in the present invention include polyamines including, but not limited to, spermidine and spermine, polycations having two or more different positively charged amino acids or basic proteins. In a preferred embodiment, the condensing agent is a polycationic polymer. In another preferred embodiment, condensing agents other than cationic lipids are used. Those of skill in the art will be aware of other condensing agents suitable for use in the present invention.

A particularly preferred example of a polycationic polymer is polyethylenimine. Polyethylenimine, which is a polymeric substance wherein every third atom is an amino nitrogen that can be protonated, has the general formula:

$$(\text{-NH-CH}_2\,\text{CH}_2\text{-})\text{-}_x[\text{-N(CH}_2\text{CH}_2\text{NH}_2)\text{CH}_2\text{CH}_2\text{-}]_y \qquad\qquad \text{I}$$

In formula I, x is approximately 2 times the value of y. Polyethylenimine is a highly branched material where the ratio of primary to secondary to tertiary nitrogens is about 1:2:1. The primary nitrogens equal the tertiary nitrogens because each branch point has a chain end. Polyethylenimine of various molecular weights can be used. Preferably, molecular weights between 0.8 kDa to about 800 kDa can be used. More preferably, a molecular weight of about 25 kDa can be used. It will be apparent to those of skill in the art that various molecular weight polymers of polyethylenimine will be suitable for use in the present invention. Polyethylenimine of various molecular weights is commercially available from Aldrich Chemical Co., (Milwaukee, Wisconsin).

The nucleic acids of this invention are typically nucleotide polymers having from 10 to 100,000 nucleotide monomers. The nucleic acids are administered to a subject for the purpose of repairing or enhancing the expression of a cellular protein. Additionally, the nucleic acid can carry a label, *e.g*., radioactive label, fluorescent label or colorimetric label for the purpose of providing clinical diagnosis relating to the presence or absence of complementary nucleic acids. Accordingly, the nucleic acids, or nucleotide polymers, can be polymers of nucleic acids including genomic DNA, cDNA, mRNA or oligonucleotides containing nucleic acid analogs, for example, the antisense derivatives described in a review by Stein, *et al., Science,* **261**:1004-1011(1993) and in U.S. Patent Nos. 5,264,423 and 5,276,019. Still further, the nucleic acids can encode transcriptional and translational regulatory sequences including promoter sequences and enhancer sequences.

The nucleotide polymers can be single-stranded DNA or RNA, or double-stranded DNA or DNA-RNA hybrids. In addition, nucleic acid with chemically modified phosphodiester bonds (*e.g.*, thiophosphodiester) are also suitable. Examples of double-stranded DNA include structural genes, genes including control and termination regions, and self-replicating systems, such as plasmid DNA. In preferred embodiments, the nucleic acid is a plasmid.

Single-stranded nucleic acids include antisense oligonucleotides (complementary to DNA and RNA), ribozymes and triplex-forming oligonucleotides and oligonucleotides having modified chemical backbones. These modifications will preferably have some or all of the nucleotide linkages substituted with stable, non-phosphodiester linkages, including, but not limited to, phosphorothioate, phosphorodithioate, phosphoroselenate, or O-alkyl phosphotriester linkages.

The nucleic acids used in the present invention will also include those nucleic acids in which modifications have been made in one or more sugar moieties and/or in one or more of the pyrimidine or purine bases. Examples of sugar modifications include replacement of one or more hydroxyl groups with halogens, alkyl groups, amines, azido groups or functionalized as ethers or esters. Additionally, the entire sugar may be replaced with sterically and electronically similar structures, including aza-sugars and carbocyclic sugar analogs. Modifications in the purine or pyrimidine base moiety include, for example, alkylated purines and pyrimidines, acylated purines or pyrimidines, or other heterocyclic substitutes known to those of skill in the art.

Multiple genetic sequences can be also be used in the present methods. Thus, the sequences for different proteins may be located on one strand or plasmid. Promoter, enhancer, stress or chemically-regulated promoters, antibiotic-sensitive or nutrient-sensitive regions, as well as therapeutic protein encoding sequences, may be included as required. Non-encoding sequences may be also be present, to the extent that they are necessary to achieve appropriate expression.

The nucleic acids used in the present method can be isolated from natural sources, obtained from such sources as ATCC or GenBank libraries or prepared by synthetic methods. Synthetic nucleic acids can be prepared by a variety of solution or solid phase methods. Generally, solid phase synthesis is preferred. Detailed descriptions of the procedures for solid phase synthesis of nucleic acids by phosphite-triester, phosphotriester, and H-phosphonate chemistries are widely available. *See,* for example, Itakura, U.S. Pat. No. 4,401,796; Caruthers *et al.,* U.S. Pat. Nos. 4,458,066 and 4,500,707; Beaucage *et al., Tetrahedron Lett.* **22**:1859-1862 (1981); Matteucci *et al., J. Am. Chem. Soc.* **103**:3185-3191 (1981); Caruthers *et al., Genetic Engineering* **4**:1-17 (1982); Jones, chapter 2, Atkinson *et al.*, chapter 3, and Sproat *et al.*, chapter 4, in *Oligonucleotide Synthesis: A Practical Approach, Gait* (ed.), IRL Press, Washington, D.C. (1984); Froehler *et al., Tetrahedron Lett.* **27**:469-472 (1986); Froehler *et al., Nucleic Acids Res.,* **14**:5399-5407 (1986); Sinha *et al. Tetrahedron Lett.* **24**:5843-5846(1983); and Sinha *et al., Nucl. Acids Res.,* **12**:4539-4557 (1984).

In addition, the nucleic acids of this invention can be chosen from among the following:

(a) gene markers, such as luciferase gene, β-galactosidase gene, chloramphenicol acetyl transferase gene, genes bestowing the resistance to an antibiotic, such as hygromycin or neomycin;

(b) genes for therapeutic purposes, such as gene encoding low density lipoprotein receptors, deficient in the case of hypercholesterolomia (liver), coagulation factors: factors VII and IX, phenylalanine-hydroxylase (phenylketonuria), adenosine deaminase (ADA immunodeficiency), lysosomic enzymes, such as β-glucosidase in the case of Gaucher's disease, dystrophine and minidistriphine (myopathy), tyrosine hydroxylase (Parkinson), neuron growth factors (Alzheimer), CFTR cystic fibrosis transmembrane conductance regulator (mucoviscidose), alphal-antitrypsin, nuclear factors: NF-KB, CII TA, cytokines and interleukines, TNF: tumor necrosis factor, thymidine kinase of the Herpes simplex virus, NO synthase, angiotensin II receptors, gene suppressors of tumors, such as the gene for the p53 protein, MHC proteins, major histocompatibility system, in particular HLA-B7, antioncogenes: p53, RB, cytosine desaminase, sense and antisense RNA; and

(c) genes with vaccine purposes: genes encoding viral antigens, for example, the nucleoprotein of the influenza virus.

Other suitable nucleic acid for use in the present invention will be readily apparent to those of skill in the art.

### C. Preparation of the Condensing Agent-Nucleic Acid Complex

Nucleic acid condensation by polycations is a function of the nature and concentration of all ions present in the condensing media. The complexation is dependent, therefore, on pH, volume and salt concentration of the complexation medium. Cationic polymer condensation with the negatively charged nucleic acid is a cooperative process that can be modulated and even inhibited in high salt concentration.

In addition, it was previously noted in O. Boussif *et al., Proc. Natl. Acad. Sci.* **92**:7297-7301 (1995), that for the cationic polymer polyethylenimine, the order of adding reagents influences the properties of the resulting particles. For instance, adding the PEI solution dropwise to the nucleic acid, *e.g.,* DNA, solution was 10-fold more efficient than adding nucleic acid to PEI.

The following method illustrates the preparation of a stock cationic polymeric solution using PEI as an example. The cationic polymer, *e.g.*, PEI, is dissolved in deionized water and neutralized to pH 7.4 with, for example, HCl. The neutralized solution is then filtered using a Millipore filter having a pore size of about 0.2 μm. In order to encapsulate the PEI/nucleic acid complex in a liposome (described hereinbelow), a small uniform particle size is critical. Large and heterogenic aggregates are a result of complexation of PEI and nucleic acid using either too high a nucleic acid concentration or solutions other than water.

An important criteria for the condensing agent-nucleic acid complex is the calculation of charge ratio. In one embodiment, the condensing agent-nucleic acid complex bears a net positive charge. A condensing agent-nucleic acid charge ratio of about 10:1 to about 2:1 is preferred, and a ratio of about 7:1 to about 4:1 is more preferred. For the PEI-nucleic acid complexes of this invention, an average mass per negative charge ratio of 325 dalton was used for plasmid DNA. The mass per positive charge for PEI was calculated to be 258 dalton. This assumes that one out of six PEI nitrogens is protonated under physiological conditions, and that the average mass per $-CH_2CH_2NH-$ repeat nitrogen unit in PEI is 43.

In another embodiment, the condensing agent-nucleic acid complex is neutral. In this embodiment, the positive charge of the condensing agent is equal to the negative charge of the nucleic acid. This results in a neutral complex.

Thus, in another embodiment, the present invention relates to a method of condensing a nucleic acid with a condensing agent to give uniformed complexes having a typical size of between about 30 nm to about 60 nm. Using PEI as atypical cationic polymer, the method involves, first, precondensing the nucleic acid by the dropwise addition of a PEI solution (10 µg of stock PEI in 250 mL water) into a nucleic acid solution (100 µg/250 mL) while vortexing Second, precondensed nucleic acid is saturated with excess PEI. Next, the PEI/nucleic acid complexes are concentrated by dialysis. Finally, the concentrated PEI-nucleic acid complexes are dialyzed overnight against HBS buffer to adjust the salt concentration to 150 mM. In the final step, other buffers can be used. These buffers include, but are not limited to, PBS, sucrose, water or organic solvent in ethanol, with the ethanol not exceeding 60-70%.

The polyethylenimine:nucleic acid ratio in the complex is about 10:1 wt/wt to about 1.5:1 wt/wt, preferably about 6:1 wt/wt to about 1.5:1 wt/wt, and, more preferably, about 4:1 wt/wt.

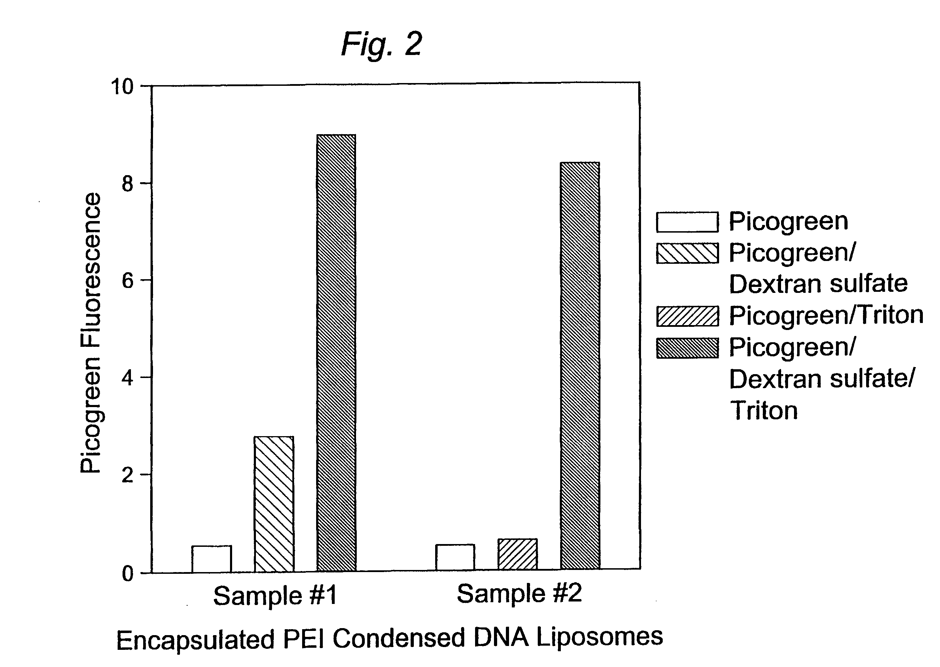

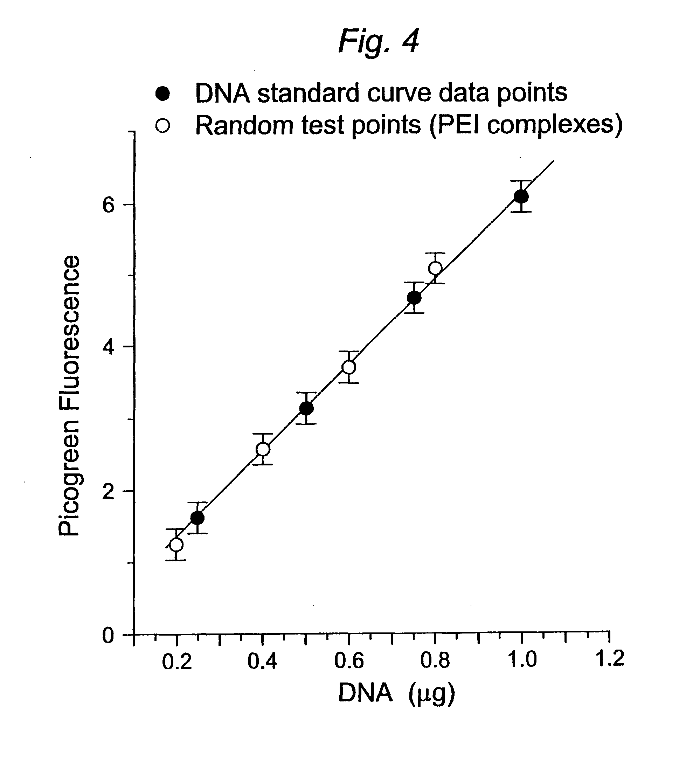

It is possible to quantitate the amount of nucleic acid condensed to the polycationic polymer. For instance, at a PEI:nucleic acid wt/wt ratio of 4:1, the complex is tightly condensed and not readily accessible to nucleic acid quantification probes like picogreen. If the nucleic acid is free and not complexed to the condensing agent, picogreen will bind to the nucleic acid and its fluorescence allows quantification of the nucleic acid. To free the nucleic acid, the complex is treated with a polyanion polymer such as dextran sulfate. Heparin, or heparan sulfate, which will "open" up or relax the complex from its condensed state can also be used. This reaction takes typically 10-15 minutes to complete. Picogreen is then added to quantify the exposed nucleic acid. A nucleic acid standard curve is set up with the range between 0.2 µg to 1 µg (*see,* Fig 4.). At each point, a standard amount of dextran sulfate is added. This addition is to offset the quenching effect dextran sulfate has on the fluorescence readings of the picogreen. This quantity is the same amount used to dissociate a PEI/nucleic acid sample. In this way, the nucleic acid associated with the polycatonic polymer can be quantitated (*see,* Fig 2). In Figure 2, the clear bar in Samples 1 and 2 represents the picogreen fluorescence *i.e.,* background.

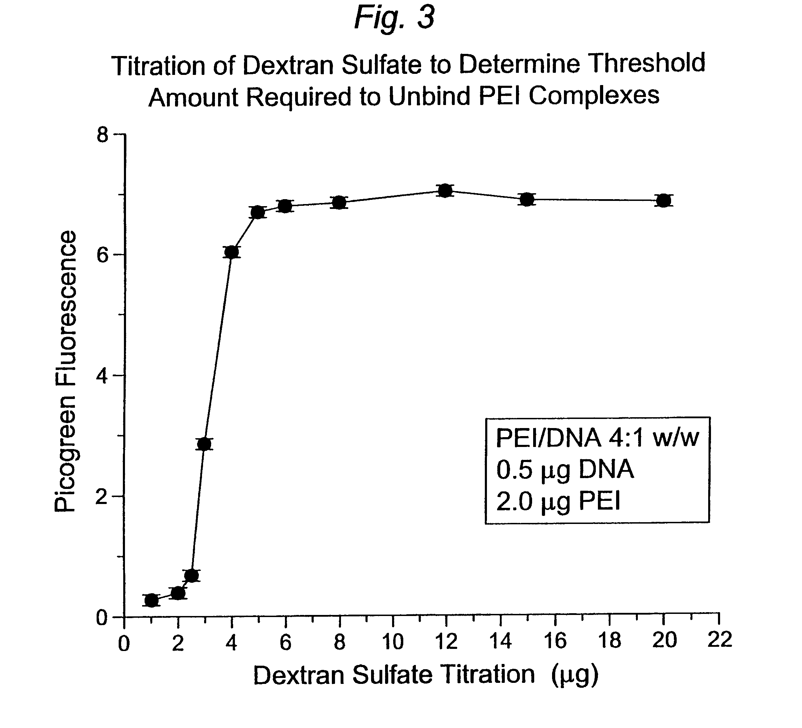

Figure 3 illustrates the titration of dextran sulfate to determine the minimal amount of dextran sulfate required to release the condensed PEI-nucleic acid complex and completely expose the complexed nucleic acid. This allows accurate quantification of the nucleic acid using a picogreen assay. At least three times more dextran sulfate than PEI is required to completely expose the DNA to picogreen. This represents a charge ratio for PEI/nucleic acid of approximately 5:1. Various charge ratios of PEI/nucleic acid complexes require differing amounts of dextran sulfate. In this manner, the charge ratio can be calculated.

## D. Encapsulation of the Condensing Agent-Nucleic Acid Complex

In another embodiment, the present invention relates to a method for encapsulating a condensing agent-nucleic acid complex in a liposome, said method comprising: adding a condensing agent solution into a nucleic acid solution to form a condensing agent-nucleic acid complex; and adding said condensing agent-nucleic acid complex to a lipid suspension to form an encapsulated condensing agent-nucleic acid complex. In a preferred embodiment, the method comprises:

(a) admixing a condensing agent solution into a nucleic acid solution to form precondensed nucleic acid;
(b) adding the precondensed nucleic acid into a condensing agent solution to form condensing agent-nucleic acid complex;
(c) dialyzing the condensed nucleic acid complex to form concentrated condensing agent-nucleic acid complex;
(d) adding said concentrated condensing agent-nucleic acid complex to a lipid suspension in a detergent; and
(e) removing said detergent from the lipid suspension to form an encapsulated condensing agent-nucleic acid complex in the liposome.

Liposomal encapsulation of the condensing agent-nucleic acid complex offers protection against enzymatic digestion and gives consistently higher gene expression than other transfer methods. To optimize the transfection capability of condensing agent-nucleic acid complexes, the overall charge of the complexes needs to be positive.

Unfortunately, with large positive charge ratios, the complexes are toxic and do not last very long in circulation. Thus, it has now been discovered that encapsulation of the condensing agent-nucleic acid complexes in liposomes can reduce the toxicity level of the complexes down to acceptable values.

A variety of lipids can be used in the liposomes of the present invention. Preferably, non-cationic lipids are used. Such lipids include, but are not limited to, phosphatidylethanolamines, phosphatidylserines, ceramides and mixtures thereof. These include, for example, dioleoylphosphatidylethanolamine (DOPE), dioleoylphosphatidylserine (DOPS) and mixtures thereof. Other examples of preferred anionic lipids suitable for use in the present invention include, but are not limited to, cardiolipin, diacylphosphatidic acid, N-succinyl-phosphatydylethanolamine (N-succinyl-PE), phosphatidic acid, phosphatidylinositol, phosphatidylglycerol, phosphatidyl ethylene glycol and mixtures thereof.

Phosphatidylethanolamines and phosphatidylserines containing saturated or unsaturated fatty acids with carbon chain lengths in the range of about $C_6$ to $C_{24}$ are preferred. Fatty acids with carbon chain lengths in the range of about $C_{14}$ to $C_{20}$ are especially preferred. Phosphatidylethanolamines with mono- or di-unsaturated fatty acids and mixtures of saturated and unsaturated fatty acids can also be used. Suitable phosphatidylethanolamines include, but are not limited to, dimyristoylphosphatidylethanolamine (DMPE), dipalmitoylphosphatidylethanolamine (DPPE), dioleoylphosphatidylethanolamine (DOPE) and distearoylphosphatidylethanolamine (DSPE). Dioleoylphosphatidylethanolamine is a preferred phosphatidylethanolamine. The preferred phosphatidylserine is dioleoylphosphatidylserine.

Ceramides suitable for use in accordance with the present invention are commercially available from a number of sources. In addition, ceramides can be isolated, for example, from egg or brain using well-known isolation techniques or, alternatively, they can be synthesized using the methods and techniques disclosed in U.S. Patent No. 5,820,873. Using the synthetic routes set forth in the foregoing application, ceramides having saturated or unsaturated fatty acids with carbon chain lengths in the range of $C_6$ to $C_{24}$ can be prepared. Preferred ceramides have acyl chain lengths of about $C_{14}$ to about $C_{20}$.

Phosphatidylethanolamines having a variety of acyl chain groups of varying chain lengths and degrees of saturation can be conjugated to polyethyleneglycol to form the bilayer stabilizing component. Such phosphatidylethanolamines are commercially available, or can be isolated or synthesized using conventional techniques known to those of skill in the art.

Ceramides having a variety of acyl chain groups of varying chain lengths and degrees of saturation can be coupled to polyethyleneglycol to form the bilayer stabilizing component. It will be apparent to those of skill in the art that in contrast to the phosphatidylethanolamines, ceramides have only one acyl group which can be readily varied in terms of its chain length and degree of saturation.

In addition, the liposome contains a bilayer stabilizing component. Examples of suitable bilayer stabilizing components include, but are not limited to, lipid, lipid-derivatives, detergents, polyethylene glycol (PEG), proteins, peptides, polyamide oilgomers, *(e.g.,* ATTA) and pH sensitive oilgomer (*e.g*., PEAA). In a presently preferred embodiment, the bilayer stabilizing component is polyethyleneglycol conjugated to, *i.e.,* coupled to, a phosphatidylethanolamine or phosphatidylserine. In an equally preferred embodiment, the bilayer stabilizing component is polyethyleneglycol conjugated to a ceramide. Polyethyleneglycol can be conjugated to a phosphatidylethanolamine, phosphatidylserine or, alternatively, to a ceramide using standard coupling reactions known to and used by those of skill in the art. In addition, preformed polyethylene-glycol phosphatidylethanolamine conjugates are commercially available from Avanti Polar Lipids (Alabaster, Alabama).

Polyethyleneglycols of varying molecular weights can be used to form the bilayer stabilizing components of the present invention. Polyethyleneglycols of varying molecular weights are commercially available from a number of different sources or, alternatively, they can be synthesized using standard polymerization techniques well-known to those of skill in the art. In a presently preferred embodiment, the polyethylene glycol has a molecular weight ranging from about 550 to about 8500 daltons, and even more preferably from about 2000 to about 5000 daltons. Generally, it has been found that increasing the molecular weight of the polyethyleneglycol reduces the concentration of the bilayer stabilizing component required to achieve stabilization.

In addition to the foregoing, polyamide oilgomers, (*e.g*., ATTA), pH sensitive oilgomers, (*e.g*., PEAA), detergents, proteins and peptides can be used as bilayer stabilizing components. Detergents which can be used as bilayer stabilizing components include, but are not limited to, Triton X-100, deoxycholate, octylglucoside and lysophosphatidylcholine. Proteins which can be used as bilayer stabilizing components include, but are not limited to, glycophorin and cytochrome oxidase. Cleavage of the protein, by endogenous proteases, resulting in the loss of the bulky domain external to the bilayer would be expected to reduce the bilayer stabilizing ability of the protein. In addition, peptides which can be used as bilayer stabilizing components include, for example, the pentadecapeptide, alanine-(aminobutyric acid-alanine)14. This peptide can be coupled, for example, to polyethyleneglycol, which would promote its transfer out of the bilayer. Alternatively, peptides such as cardiotoxin and melittin, both of which are known to induce non-lamellar phases in bilayers, can be coupled to PEG and might thereby be converted to bilayer stabilizers.

Typically, the bilayer stabilizing component is present at a concentration ranging from about 0.05 mole percent to about 50 mole percent. In a presently preferred embodiment, the bilayer stabilizing component is present at a concentration ranging from 0.05 mole percent to about 25 mole percent. In an even more preferred embodiment, the bilayer stabilizing component is present at a concentration ranging from 5 mole percent to about 15 mole percent. One of ordinary skill in the art will appreciate that the concentration of the bilayer stabilizing component can be varied depending on the bilayer stabilizing component employed.

One method of encapsulating the complexes of this invention is by using detergent dialysis. Typically, the encapsulation of the complexes is accomplished by dissolving the lipids in a solvent and then drying the solution under a stream of nitrogen. Preferably the lipids are non-cationic lipids. More preferably, the lipids are DOPE, DOPS, PEG-ceramide and mixtures thereof. The ratio of lipid to nucleic acid is about 5:1 wt/wt to about 100:1 wt/wt, preferably about 10:1 wt/wt to about 50:1 wt/wt. Final total lipid concentration desired is about 10 mg/mL. A thin lipid film is achieved by including a mixing step, such as vortexing, in the drying procedure. Any remaining solvent is removed by freeze-drying.

The lipid film is then dissolved in a detergent, or alternatively, ethanol. The condensing agent-nucleic acid complex is then added, such as a PEI/nucleic acid complex having a nucleic acid concentration of about 50 μg/mL to about 1000 μg/mL. A nucleic acid concentration of about 400 μg/mL is preferred. The resulting mixture is then vortexed until it becomes clear and then dialyzed. This procedure results in a liposome encapsulating the condensing agent-nucleic acid complex. The method can be scaled up proportionately for use in larger preparations.

The detergents that are useful for encapsulating the condensing agent-nucleic acid complexes in the present invention are typically one or more neutral detergents or combinations of detergents and organic solvents. The detergents are preferably, N,N'-((octanoylimino)-bis-(trimethylene))-bis-(D-gluconamide) (BIGCHAP); BRIJ 35; Deoxy-BIGCHAP; dodecylpoly(ethylene glycol) ether; Tween 20; Tween 40; Tween 60; Tween 80; Tween 85; Triton X-405; hexyl-, heptyl-, octyl- and nonyl-β-D-glucopyranoside; with octyl β-D-glucopyranoside being the most preferred.

The organic solvents that are useful in combination with a detergent include, but are not limited to, chloroform, dichloromethane, diethylether, cyclohexane, cyclopentane, benzene, toluene, acetone, benzyl alcohol, methanol, or other aliphatic alcohols such as propanol, iso-propanol, butanol, *tert*-butanol, iso-butanol, pentanol and hexanol. The selection of an organic solvent will typically involve consideration of solvent polarity and the ease with which the solvent can be removed at the later stages of encapsulation. Accordingly, the preferred organic solvents used in conjunction with the detergent are ethanol, dichloromethane, chloroform, methanol and diethyl ether with chloroform and methanol being the most preferred.

The solution of non-cationic lipids, bilayer stabilizing component and detergent is an aqueous solution. Contacting the condensing agent-nucleic acid complex with the solution of non-cationic lipids and detergent is typically accomplished by mixing together a first solution of nucleic acids and a second solution of the lipids and detergent. One of skill in the art will understand that this mixing can take place by any number of methods, for example, by mechanical means such as by using vortex mixers. Preferably, the nucleic acid solution is also a detergent solution.

In an alternative embodiment, a dehydration-rehydration method can be used to encapsulate the condensing agent-nucleic acid complex. In this method, a lipid mixture in a solvent is dried down and then rehydrated in a buffer containing the condensing agent-nucleic acid complex. Extrusion of the liposome follows the rehydration step. The dehydration-rehydration method generates lower encapsulation efficiency than the detergent dialysis technique described above.

In yet another embodiment, the reverse phase evaporation method can be employed to encapsulate the complex. In this method, the lipids are first dissolved in a solvent system or mixed solvent system. The condensing agent-nucleic acid complex is dissolved in water and then added to the lipid mixture. The solvent system is added until a single phase is observed. After excess solvent is removed by evaporation, the solution is extruded to yield encapsulated liposomes.

In still yet another embodiment, the ethanol injection method can be used for encapsulation of the complex. In this method, the lipids are dissolved in ethanol, or another suitable solvent, and dripped into a tube containing the condensing agent-nucleic acid complexes in water. The liposomes are formed immediately and the ethanol is dialyzed away to yield encapsulated liposomes.

The size of the liposomes of the present invention are about 20 nm to about 200 nm in diameter. More preferably, the liposomes of the present invention are about 50 nm to about 150 nm in diameter. In an especially preferred embodiment, the liposomes of the present invention are about 70 nm to about 80 nm in diameter.

The size distribution of the condensing agent-nucleic acid complexes and liposomes can be measured by quasielastic light scattering using a Nicomp Submicron Particle Sizer (Model 370) in the solid particle mode and vesicle mode, respectively.

To measure the encapsulation efficiency of the liposomes using the methods above, picogreen and dextran sulfate are used. The amount of unencapsulated complexes $M_{uncap}$ (determined from fluorescence of picogreen) can then be quantified via the combination of dextran sulfate and picogreen. By adding Triton X-100, which completely breaks apart the liposomes, it is also possible to quantify the total DNA present, $M_{tot}$. The extent of encapsulation is then calculated via the formula:

$$\% \text{ Encapsulation} = (1 - M_{uncap}/ M_{tot.}) \times 100$$

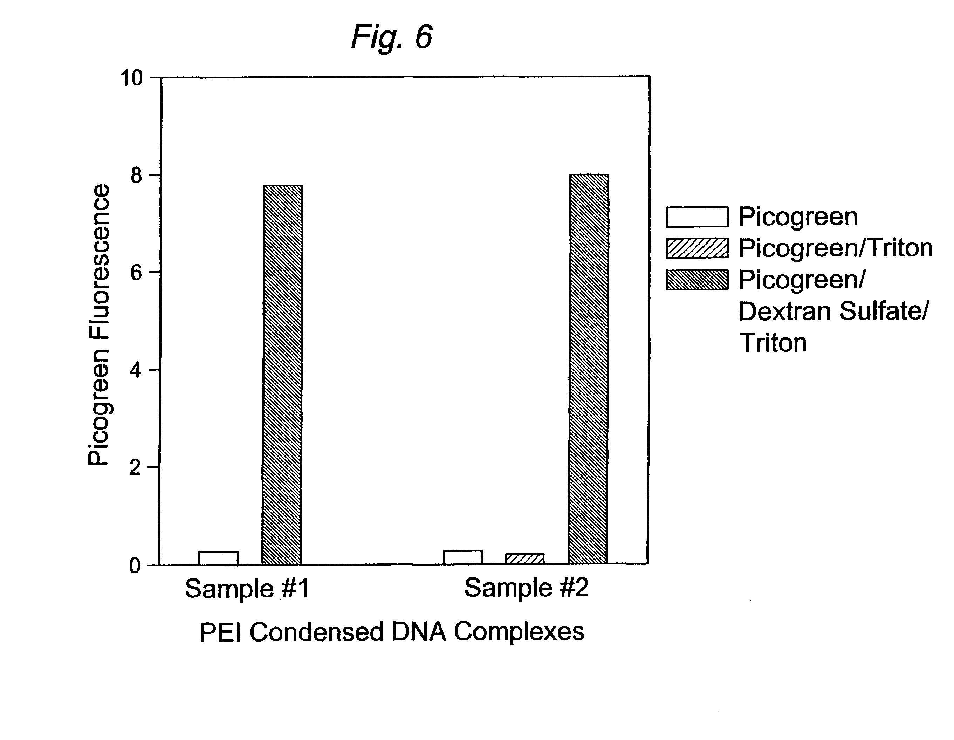

Encapsulation efficiency is best described with reference to Figure 6. As shown in Figure 6, when picogreen is added to an unencapsulated complex, fluorescence is in the background. When dextran sulfate is added to an unencapsulated complex, there is an increase in fluorescence to 3.0. When Triton X-100 is added to break up the liposomes, a further jump in fluorescence occurs (*see*, Sample 1). The ratio of these peaks gives the extent of encapsulation. To remove any unencapsulated complex, a cation exchange column can be used.

In using methods of the present invention, it is possible to encapsulate about 30% to about 70% of the condensing agent-nucleic acid complex . Preferably, percent encapsulation is about 40% to about 70% and most preferably, percent encapsulation is about 50% to about 70%.

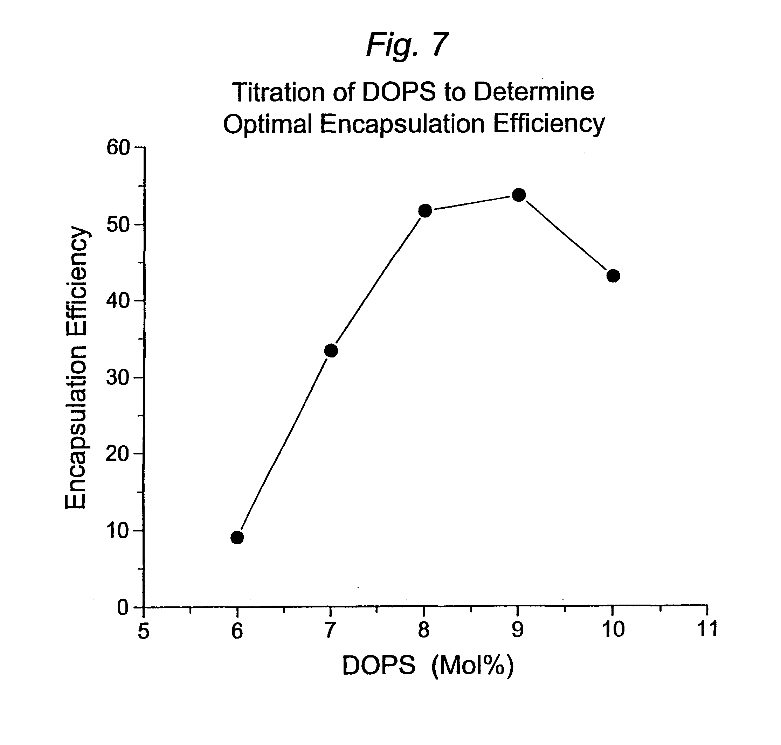

With reference to Figure 7, the titration of DOPS to optimize encapsulation efficiency is illustrated. In certain embodiments, a concentration of approximately 8-9 mol% of DOPS in the liposome gives the best entrapment of the complexes, in this case PEI/DNA. Shifting this point by more than 2% in concentration of DOPS drops the encapsulation efficiency dramatically.

### E. Administration of Liposome-Entrapped Complexes

Following formation of the liposomal entrapped condensing agent-nucleic acid complexes, the liposomes can be used to transfect cells by contacting the cells to be transfected with the liposomes. The liposome-entrapped complexes can be adsorbed to almost any cell type. Once adsorbed, the liposomes can either be endocytosed by a portion of the cells, exchange lipids with cell membranes, destabilized or fuse with the cells. Transfer or incorporation of the nucleic acid portion of the liposome can take place via any one of these pathways. In particular, when fusion takes place, the lipid bilayer membrane is integrated into the cell membrane and the contents of the bilayer combine with the intracellular fluid. Fusion of the liposome with the plasma membrane takes place when the bilayer stabilizing component transfers out of the liposome and the bilayer stability is lost or decreased. Without being bound to any theory, polycationic mediated gene transfer is thought to involve DNA aggregation and binding of the resulting complex to anionic residues on the plasma membranes. To be efficient, the complex should bear a net positive charge.

Contact between the cells and the liposomal entrapped condensing agent-nucleic acid complexes, when carried out *in vitro,* will take place in a biologically compatible medium.

Treatment of the cells with the liposome-entrapped complex will generally be carried out at physiological temperatures (about 37°C) for periods of time ranging from about 1 to 48 hours, preferably from about 2 to 4 hours. For *in vitro* applications, the delivery of nucleic acids can be to any cell grown in culture, whether of plant or animal origin, vertebrate or invertebrate, and of any tissue or type. In preferred embodiments, the cells will be animal cells, more preferably mammalian cells, and most preferably human cells.

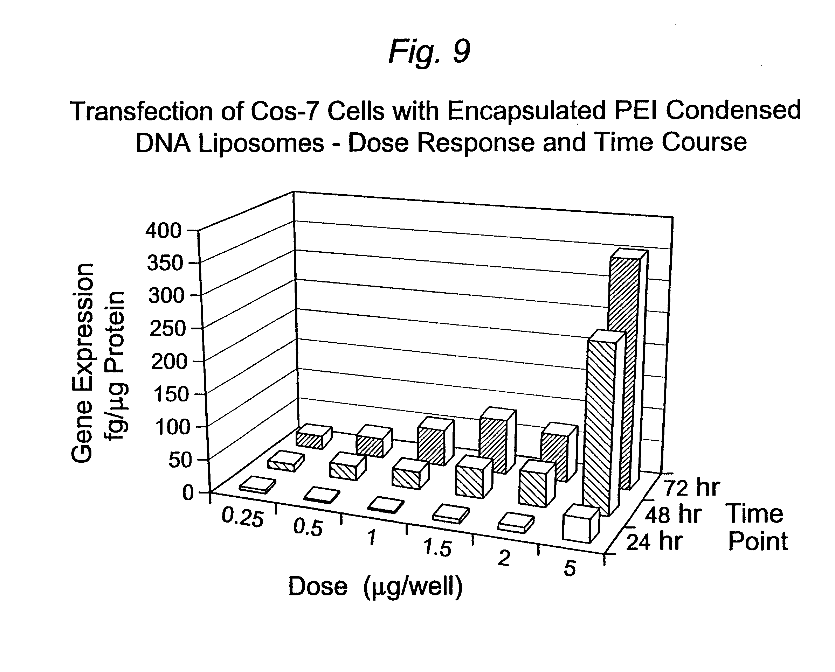

With reference to Figure 9, Cos-7 cells were transfected with 1 μg of encapsulated pINEX/L018 plasmid DNA complexed with PEI at the 1:4 w/w ratio. In a dose response and time course analyses, Figure 9 shows that transfection activity increased as DNA dose increased. The highest transfection activity was observed at 5 μg of DNA. Minimal transfection was seen at the 24 hour time point. The transfection activity continued to increase up to the 72 hour time point.

### F. Pharmaceutical Preparations

The liposome-entrapped condensing agent-nucleic acid complexes of the present invention can be administered alone or in mixture with a physiologically acceptable carrier. Such carriers include, but are not limited to, physiological saline or phosphate buffer selected in accordance with the route of administration and standard pharmaceutical practice.

Pharmaceutical compositions comprising the liposome-entrapped condensing agent-nucleic acid complexes are prepared according to standard techniques and further comprise a pharmaceutically acceptable carrier. Generally, normal saline will be employed as the pharmaceutically acceptable carrier. Other suitable carriers include, *e.g.,* water, buffered water, 0.4% saline, 0.3% glycine, and the like, including glycoproteins for enhanced stability, such as albumin, lipoprotein, globulin, *etc.* In compositions comprising saline or other salt containing carriers, the carrier is preferably added following liposome formation. Thus, after the liposome-entrapped complexes are formed, the liposome can be diluted into pharmaceutically acceptable carriers, such as normal saline. These compositions may be sterilized by conventional sterilization techniques. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, *etc*.

The concentration of the liposome-entrapped complexes in the pharmaceutical formulations can vary widely, *i.e.*, from less than about 0.05%, usually at or at least about 2-5% to as much as 10 to 30% by weight and will be selected primarily by fluid volumes, viscosities, *etc.*, in accordance with the particular mode of administration selected. For example, the concentration may be increased to lower the fluid load associated with treatment. For diagnosis, the amount of liposome-entrapped complex administered will depend upon the particular label used, the disease state being diagnosed and the judgement of the clinician but will generally be between about 0.01 and about 50 mg per kilogram of body weight, preferably between about 0.1 and about 5 mg/kg of body weight.

In another example of their use, the liposomal entrapped condensing agent-nucleic acid complexes can be incorporated into a broad range of topical dosage forms including, but not limited to, gels, oils, emulsions and the like. For instance, the suspension containing the liposomal entrapped condensing agent-nucleic acid complexes can be formulated and administered as topical creams, pastes, ointments, gels, lotions and the like.

The present invention also provides liposome-entrapped condensing agent-nucleic acid complexes in kit form. The kit will typically be comprised of a container which is compartmentalized for holding the various elements of the kit. The kit will contain the liposomes of the present invention, with instructions for administration. In still other embodiments, the liposomal entrapped condensing agent-nucleic acid complexes will have a targeting moiety attached to the liposome. Methods of attaching targeting moieties (*e.g.*, antibodies, proteins) to lipids (such as those used in the present invention) are known to those of skill in the art.

Dosage for liposome-entrapped condensing agent-nucleic acid complexes will depend on the ratio of nucleic acid to lipid and the administrating physician's opinion based on age, weight, and condition of the patient.

The invention will be described in greater detail by way of specific examples. The following examples are offered for illustration only.

### G. Examples

### I. Materials

The reporter gene plasmid used in all experiments was pINEX/L018 (5650 bp), which encodes firefly luciferase under the control of the human cytomegalovirus immediate-early enhancer/promoter. Plasmid DNA was prepared from *E. coli* DH5alpha by alkaline lysis followed by double banding on cesium chloride gradients. (*See*, Thierry A.R., *J. Liposome Research* 7:143-159 (1997)). PEI 25 kDa was purchased from Aldrich Chemical Co. (Milwaukee, WI). Ethidium bromide picogreen was obtained from Sigma Chemical Co. (St. Louis, MO). Purified firefly luciferase was purchased from Boehringer Mannheim (Germany). All lipids, with exception of the PEG-ceramide (made in-house), are obtained from Avanti Lipids. Fetal bovine serum was purchased from Intergen (New York, USA). All culture media were purchased from Stemcell Technology (Vancouver, BC). Other reagents in this study were from Sigma Chemical Co., and used without further purification.

### II. Methods

#### a. Method for Preparing PEI/plasmid DNA complexes.

1.) PEI solution (1.6 mg PEI/mL water): 0.1 volume of the PEI solution was added dropwise, using a syringe fitted with a needle size 26G3/8 gauge, into a plasmid DNA solution (1 mg plasmid DNA/1.25 mL water) while vortexing. 2.) The precondensed PEI/DNA made in step 1, was then added dropwise, using a syringe fitted with a needle size 26G3/8 gauge, into PEI solution (390 g of stock PEI diluted in 500 mL water) while vortexing to form PEI/plasmid DNA complexes. The resulting surface charge of the PEI/DNA complex is a net positive charge. 3.) The PEI/plasmid DNA complex was then transferred into a dialysis bag (6000 - 8000 molecular weight cut-off, Spectra-Por, Spectrum) and covered with a drying agent (polyethylene glycol, 10,000 molecular weight). 4.) The concentrated PEI/plasmid DNA complexes was dialyzed overnight against HBS (150 mM NaCl, 5 mM - 25 mM HEPES), pH 7.45 to adjust the NaCl concentration to 150 mM.

#### ii. Method for the Quantification of Plasmid DNA.

The PEI/plasmid DNA complexes were treated with a polyanion polymer such as dextran sulfate in water (other polymers such as heparin or heparan sulfate can also be used) where for every 4 μg PEI used, 40 μg of dextran sulfate is used. This reaction takes typically between 10 to 15 minutes to complete. 4 μl picogreen is then added to the PEI/plasmid DNA complex. A DNA standard curve is set up with a range running between 0.2 µg to 1.0 µg, where at each point, a standardized amount of dextran sulfate is added. This is to offset the quenching effect of the dextran sulfate on the fluorescence readings of the picogreen. This amount must also be the same as the amount used to dissociate the PEI/plasmid DNA sample.

<u>iii. Protocol for Encapsulating the PEI/plasmid DNA Complexes in Lipids.</u>

The lipids, DOPE (82 mol %), DOPS (8 mol %), and Peg-Ceramide(C20) (10 mol %), dissolved in chloroform, were first dried under a stream of nitrogen. Final total lipid concentration desired is 10 mg/mL. A thin lipid film is achieved by including vortexing in the drying procedure. Any remaining solvent was removed by further freeze-drying overnight. The dried-down lipid film is then removed from the lyophilizer and 200 µl of OGP (200 mM) was added. Hard vortexing, followed by warming at 65°C for 5 minutes intermittently, helps to dissolve the lipids in the detergent. When no apparent undissolved lipid can be seen, the PEI/DNA complexes in the DNA concentration of 400 µg/mL, is then added to the lipid suspension. The lipid:DNA w/w ratio used is 10mg:400 µg respectively. Anything above the concentration of 500µg/ mL DNA was found to cause precipitation or flocculation to occur (final product has big particles which disappeared momentarily upon shaking). The resulting mixture is then vortexed until it becomes clear and then transferred into a Spectra-Por dialysis bag for dialysis. The dialysis buffer is made up of 5 mM Hepes, 150 mM NaCl and titrated with appropriate amounts NaOH to achieve a pH of approximately 7.45. The mixture is then dialyzed for 24 hours with buffer changes at every 4 hours.

<u>iv. Method for Determining Encapsulation Efficiency of the Lipid Formulation.</u>

To measure the encapsulation efficiency of lipid particles, picogreen and dextran sulfate were used. The amount of unencapsulated complexes $M_{uncap}$ (determined from fluorescence of picogreen) could be quantified via the combination of dextran sulfate and picogreen. When Triton X-100 was added, which completely dissociates the lipid particles, the total DNA present, $M_{tot}$ could be determined. The extent of encapsulation is then calculated using the formula:

$$\% \text{ Encapsulation} = (1 - M_{uncap}/ M_{tot}) \times 100$$

<u>v. Protocol for treating the Encapsulated PEI/plasmid DNA Lipid Particles.</u>

The cation gel, about 100 mL (Dowex-50W, Catalog No. 50X8-400, Sigma) is first placed in a volumetric flask (1000 mL capacity) containing approximately 500 mL of 0.5 M HCl, which acts as a proton reservoir. A magnetic stir bar was included in the flask and the mixture was stirred slowly on a stir-plate overnight. The following day, the gel is then loaded into a chromatographic column (GlassEcono-column, Catalog No. 737-1012, BioRad) to a height of about 5 cm. The gel is then washed with 10 volumes of distilled water to normalize the pH of the gel. After the wash, 100 mL of 5 M NaCl is used to wash off any remaining impurities on the gel. Finally, 10 column volumes of 150 mM NaCl HBS was used to equilibrate the column.

<u>v. Method for Transfection of the PEI/plasmid DNA Lipid Particles Using Several Cell Lines *in vitro*.</u>

Lewis Lung, SK-OV-3, LS180, Cos 7, B16 and U87 cells were seeded in 24-well plates (Falcon 3047) at a density of $4 \times 10^4$, $2 \times 10^4$, $4 \times 10^4$, $2 \times 10^4$, $4 \times 10^4$ and $4 \times 10^4$ cells/well in 1 mL of media with 10% fetal bovine serum. B16 and LS180 cells were grown in MEM + Earls Salts, Lewis Lung, Cos 7 and U87 cells were grown in DMEM with high glucose, and SK-OV-3 cells were grown in RPMI 1640 medium. Cells were incubated overnight to 70-80% confluent at the time of transfection. The encapsulated PEI condensed DNA lipid formulations were prepared as described above and were added to the appropriate wells in triplicate. The plates were agitated briefly then incubated at 37°C (5% $CO_2$) for 24 and 48 hours before assaying for gene expression.
Luciferase gene expression was measured with a luminometer (Dynatech Microlite TM ML3000 Microtiter) using 96-well plates (Catalog No. 011-010-7411, Dynatech ). Cells were rinsed once with PBS buffer, and were then lysed with 150 mL of lysis buffer (0.1% Triton X-100, 250 mM sodium phosphate buffer, pH 8.0) at room temperature for 10 - 15 minutes. Duplicate assays for 10 µl of cell lysate were performed. A standard curve was prepared using purified luciferase protein diluted into mock transfected cell lysate.

vi. Method for Measuring the Total Protein of Each *in vitro* Transfected Sample

Protein assay was performed by using the bicinchoninic acid (BCA) colorimetric method. In this assay, 10 µl of lysate was transferred to the individual wells of 96-well plate (Catalog No. 011 - 010-7411, Dynatech), 200 µl of Micro BCA working reagent was added to each well, mixed and incubated at 37°C for 2 hours. The amount of protein in each well was determined by comparison with BSA protein standard (1-16 µg/well) added to a series of duplicate wells on the same plate. The plate with samples and BSA protein standard was read at 570 nm in a microtiter plate reader (Dynatech MR5000) after allowing the color to develop.

vii Method of Evaluating the Level of Toxicity *in vitro*

Dilute 0.1% crystal violet reagent to 0.05% with 20% ethanol. Centrifuge plates at 1500 rpm for 10 minutes. Rinse plates 2 times with PBS buffer by pouring the buffer into the lid of a pipette tip box and submerging the plates. Change the PBS buffer between plates. Invert plates onto a stack of paper towels and gently pat to dry. Add 50-100 µl of 0.05% crystal violet to each well. Incubate plates at room temperature for 10 minutes. Rinse plates with tap water as described above for the PBS wash. Allow plates to dry on the bench top overnight. Add 100 µl of 100% methanol to each well. Read plates within 5 minutes of methanol addition using the plate reader at 570nm.
Cos-7 cells were seeded onto 96-well plates at a density of 2.5 x $10^3$ in 200 µl of completed medium and incubated 72 hours to 90% confluence. different amount of encapsulated PEI condensed DNA formulations and PEI/DNA complex were added to the appropriate wells in triplicate. Stained the cells with crystal violet after 24 hours incubated.

viii Method for delivering the PEI/plasmid DNA lipid particles in the *in vivo* system

Female C57 mice were injected intraperiotenially (i.p.) with 1 x $10^5$ B16 tumor cells. On day 7 of B 16 tumor growth, DNA doses of 75 (g luciferase plasmid/formulation (encapsulated PEI/DNA lipid particles) were administered in a volume of 500 µl by intraperitoneal injection. Control animals were injected with the same volume of saline. Tumors were collected at different time points, fast frozen in liquid nitrogen and stored at -70°C until analysis. Individual tumors were homogenized using a FastPrep homogenizer (Bio101 inc.) for 5 seconds at speed setting of 5, loaded with a small bead (Catalog No. 6520-401/404, Bio 101), then a second bead and a certain amount of 1 x CCLR reagent (Cell Culture Lysis Reagent Catalog No. E1531, Promega) supplemented with 1 mg/mL BSA (Catalog No. A-2153, Sigma) was added to each tube. The homogenization was performed twice in the FastPrep instrument (FastPrep™ FP120 Instrument, Bio 101) using a speed setting of 5 for 6 seconds. The homogenate was transferred to fresh Eppendorf tubes and large tissue debris was pelleted to the bottom of the tube by brief centrifugation. 20 µl of homogenate and standard luciferase protein diluted with control tissue homogenate were assayed in duplicates. The results were converted to pg of luciferase protein per organ or gram of tumor.

ix. Method for Evaluating Toxicity in the *in vivo* system by Measuring Levels of Aminotransferase (AST/GOT) Activity in Serum.

1.) Add 10 mL of deionized water to a vial. Mix immediately several times by inversion (not shaking). Store reagent up to 16 hours at room temperature or up to 7 days refrigerated. 2.) Turn the UV lamp on at least one half an hour before analysis. Go to kinetics analysis and use the default program within the kinetics setting. Set spectrophotometer to 340 nm and the times as 30, 60, 90, and 120 seconds. Blank with water. 3.) Add 500 µl of reagent to the cuvette (make sure the reagent is at 25°C). 4.) Add 50 µl of the test serum and mix by pipetting up and down. 5.) Read the absorbance after 60 seconds. This is the initial value (inA). 6.) Read the absorbance 30 seconds after the inA reading. This reading is used to verify a linear reaction. 7.) Read the absorbance 60 seconds after the inA reading. This is the final reading (finA). 8.) Calculate the (A per minute by subtracting FinalA from InitialA. If (A per minute is greater than 0.280, dilute 1 part sample with 1 part isotonic saline and reassay. Multiply the results by 2 to compensate for the dilution. 9.) To calculate the

$$AST(U/L) = \frac{(A \text{ per minute x TV x 1000}}{6.22 \text{ x LP x SVTV}} = \text{Total volume (0.55 mL)}$$

SV =          Sample volume (0.55 mL)
6.22 =        Millimolar absorptivity of NADH at 340 nm
LP =          Light path (1.0)
1000 =        Conversion of units per mL to units per liter

$$= \frac{\text{(A per minute x 0.55 x 1000)}}{6.22 \times 1.0 \times 0.05}$$
$$= \text{(A per minute x 1786 (x 1.37 if values were determined at 25°C)}$$

One unit of activity is defined as the amount of enzyme which produces 1 (mole of NAD per minute under the conditions of the assay procedure.

EXAMPLE 1

This example illustrates the effect of dextran sulfate on PEI/DNA complexes.

Figure 2 (sample 1) illustrates that when picogreen is added to a sample of the liposome formulation containing a PEI/DNA complex, the fluorescence reading is well in the background. It is clear that the DNA is well protected by the condensing agent PEI. When dextran sulfate is added, a significant jump in fluorescence results. The DNA is clearly not in the same condensed form, thus allowing access to picogreen. In sample 2, when Triton X100 is added, there is no significant change in the fluorescence reading *i.e.*, similar to background. This is an indication that Triton does not affect the complexes in any significant way. Upon addition of dextran sulfate, the fluorescence increases.

EXAMPLE 2

This example illustrates the use of dextran sulfate and the amount required for complex dissociation

As is illustrated in Figure 3, when the amount of dextran sulfate added is increased, the fluorescence also increases. This indicates that more and more DNA is made accessible to the picogreen. The increase in fluorescence eventually tapers off at some level and eventually the fluorescence signal is quenched by the excess dextran sulfate. The optimum dextran sulfate to PEI w/w ratio is around 6:1. It is noted that each point is Figure 3 includes an incubation period of approximately 15 minutes.

EXAMPLE 3

This example illustrates the relaxation time of the PEI-DNA complexes.

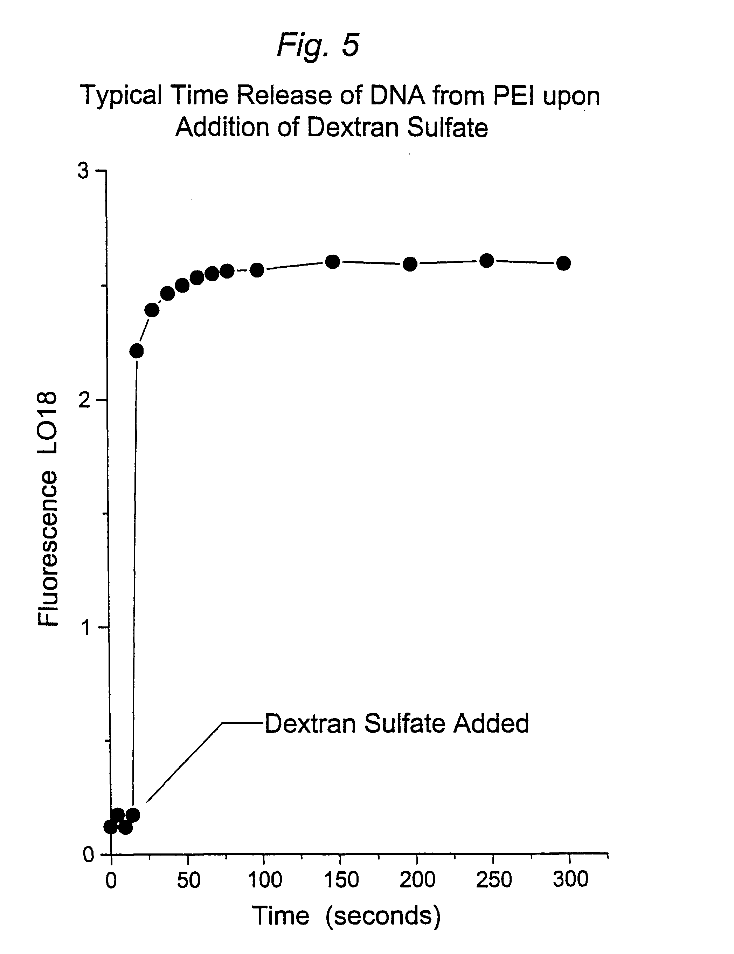

Figure 5 illustrates the time relaxation profile of PEI/DNA complexes under the effect of dextran sulfate. The graph shows clearly that the relaxation of the complexes is not an instantaneous event. The initial relaxation is rapid, slowing down eventually to final equilibration, as indicated by the picogreen fluorescence signal. These results indicate that for quantification purposes, the required amount of dextran sulfate must be added in advance to the sample and allowed to incubate for at least 15 minutes, to ensure that the relaxation process is complete.

EXAMPLE 4

This example illustrates the encapsulation efficiency of PEI/DNA complexes into liposomes.

As shown in Figure 7, to optimize the encapsulation efficiency, DOPS was titrated and it was found that between 8-9 mol% DOPS gives the best encapsulation at about 55%. This percent encapsulation was determined prior to column loading. Encapsulation efficiencies drop dramatically below this concentration, indicating the high sensitivity of the procedure on the negative surface-charge density. Note that this was obtained in a buffer with 150 mM NaCl concentration. Changing the NaCl concentration will change the amount of DOPS required to optimize encapsulation. For *in vitro* tests, about 8 mol % of DOPS was optimum.

EXAMPLE 5

This example illustrates the efficiency of transfection of the PEI/DNA complexes *in vitro.*

As shown in Figure 9, Cos-7 cells were transfected with 1 μg of encapsulated pINEX/L018 plasmid DNA complexed with PEI at a 1:4 w/w ratio. In this experiment, dose response and time course were analyzed. Figure 9 shows that activity increased as DNA dose increased. The highest transfection activity was observed at 5 μg of DNA. Minimal transfection was seen at the 24 hour time point. The transfection activity continued to increase up to the 72 hour time point.

EXAMPLE 6

This example illustrates the reduction of toxicity of the encapsulated PEI/DNA complexes.

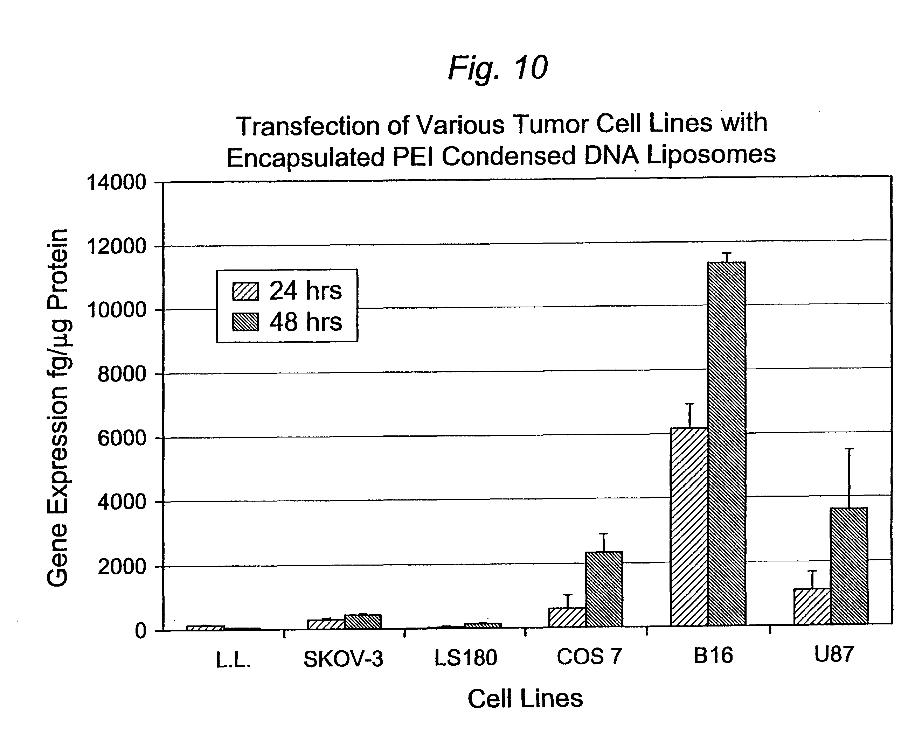

As shown in Figure 10, a toxicity study was conducted of an encapsulated PEI/DNA complex with a dose response having a complex charge-ratio at 5.3. As an illustration, the time-point selected was 48 hours. The control was the cell line with no added components. The graph clearly shows that unencapsulated complexes shows significant toxicity beginning at the I µg dose. The encapsulated complexes showed no relative toxicity up to 2 µg DNA.

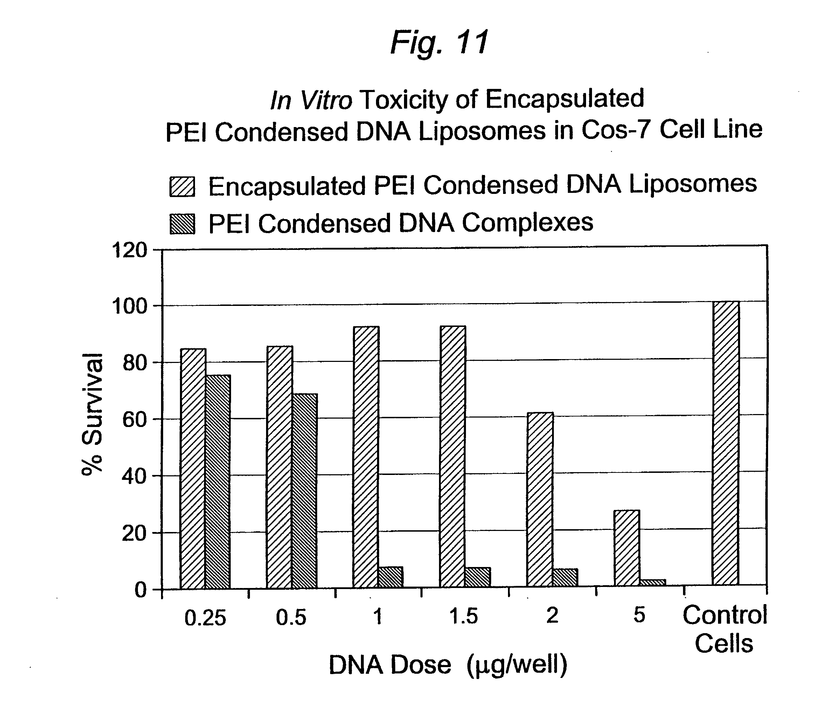

As shown in Figure 11, the comparison of toxicity between (1) liposomes not treated by a cation exchange column and (2) those which have been so treated is illustrated. The unencapsulated complexes contains 0.75 µg DNA, which is selected to be exactly what is known to be on the outside of the liposome sample. The toxicity of (1) is seen to be comparable to that of the unencapsulated complexes. Sample (2) shows dramatic reduction in toxicity, and can be attributed to the removal of the complexes by a cation exchange column.

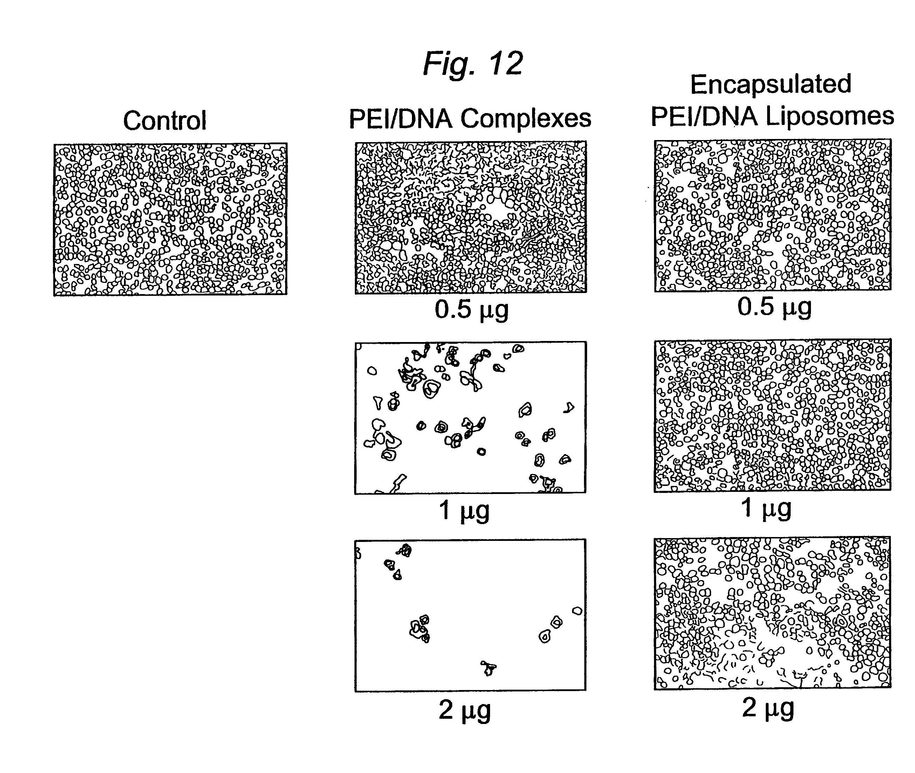

Figure 12 illustrates *in vivo* toxicity of encapsulated PEI/DNA complexes. Encapsulated PEI/DNA with a dose of 75 µg DNA is injected into 4 mice and the enzyme (AST) levels were found to be comparable to that of the PBS control. At a dosage of about 4:1 w/w ratio of PEI/DNA, an unencapsulated sample would have been lethal to the mice. The encapsulation efficiency in this liposomal injection is close to 90%.

## Claims

1. A liposome comprising:

    (a) a lipid; and
    (b) a condensing agent-nucleic acid complex encapsulated in the liposome.

2. A liposome according to claim 1, wherein the lipid comprises a non-catonic lipid.

3. A liposome according to claim 1 or claim 2, wherein the condensing agent is selected from polyethylenimines, polylysines, polyarginines, polyomithines, histones, protamines, polyamines, spermidine and spermine.

4. A liposome according to claim 3, wherein the condensing agent is a polyethylenimine having a molecular weight of 0.8 to 800 kDa.

5. A liposome according to any one of claim 4, wherein the polyethylenimine:nucleic acid weight ratio in the condensing agent-nucleic acid complex is 10:1 to 1.5:1.

6. A liposome according to any one of claims 1 to 5, wherein the condensing agent-nucleic acid complex has a diameter of 30 to 60 nm.

7. A liposome according to any one of claims 1 to 6, having a diameter of 20 to 200 nm.

8. A liposome according to claim 7, having a diameter of 50 to 150 nm.

9. A liposome according to claim 8, having a diameter of 70 to 80 nm.

10. A liposome according to any one of claims 1 to 9, further comprising:

    (c) a bilayer stabilizing component associated therewith.

11. A liposome according to claim 10, wherein the bilayer stabilizing component is selected from lipids, lipid-derivatives. detergents, polyethylene glycols, proteins, peptides, polyamide oligomers, pH-sensitive polymers and PEG-lipids.

12. A liposome according to claim 11, wherein the PEG-lipid is a PEG-ceramide.

13. A liposome according to claim 12, wherein the PEG has an average molecular weight of 2000 to 5000 Daltons.

14. A liposome according to any one of claims 1 to 3, wherein the encapsulated condensing agent-nucleic acid complex represents greater than about 30% encapsulation efficiency as determined using picogreen and dextran sulfate.

15. A method of transfecting a cell with a nucleic acid in vitro, the method comprising contacting the cell with a liposome according to any one of claims 1 to 14.

16. A method for encapsulating a condensing agent-nucleic acid complex in a liposome, the method comprising:

adding a condensing agent solution into a nucleic acid solution thereby to form a condensing agent-nucleic acid complex; and

adding the condensing agent-nucleic acid complex to a lipid suspension thereby to form an encapsulated condensing agent-nucleic acid complex.

17. A method according to claim 16, wherein the condensing agent-nucleic acid complex is formed by admixing a first condensing agent thereby to form a precondensed nucleic acid and then adding the precondensed nucleic acid into a second condensing agent solution thereby to form the condensing agent-nucleic acid complex and wherein the first and the second condensing agents are the same or different.

18. Use of a liposome according to any one of claims 1 to 14 for the manufacture of a medicament for transfecting a cell.

**Patentansprüche**

1. Liposom, welches enthält:

   (a) ein Lipid und
   (b) einen in dem Liposom eingekapselten Komplex aus einem Kondensationsmittel und Nukleinsäure.

2. Liposom nach Anspruch 1, wobei das Lipid ein nichtkationisches Lipid umfaßt.

3. Liposom nach Anspruch 1 oder Anspruch 2, wobei das Kondensationsmittel unter Polyethyleniminen, Polylysinen, Polyargininen, Polyornithinen, Histonen, Protaminen, Polyaminen, Spermidin und Spermin ausgewählt ist.

4. Liposom nach Anspruch 3, wobei das Kondensationsmittel ein Polyethylenimin mit einem Molekulargewicht von 0,8 bis 800 kDa ist.

5. Liposom nach Anspruch 4, wobei das Gewichtsverhältnis von Polyethylenimin zu Nukleinsäure in dem Komplex von Kondensationsmittel und Nukleinsäure 10:1 bis 1,5:1 beträgt.

6. Liposom nach einem der Ansprüche 1 bis 5, wobei der Komplex von Kondensationsmittel und Nukleinsäure einen Durchmesser von 30 bis 60 nm hat.

7. Liposom nach einem der Ansprüche 1 bis 6, das einen Durchmesser von 20 bis 200 nm hat.

8. Liposom nach Anspruch 7, das einen Durchmesser von 50 bis 150 nm hat.

9. Liposom nach Anspruch 8, das einen Durchmesser von 70 bis 80 nm hat.

10. Liposom nach einem der Ansprüche 1 bis 9, welches außerdem

    (c) eine assoziierte Komponente zum Stabilisieren einer Doppelschicht enthält.

11. Liposom nach Anspruch 10, wobei die eine Doppelschicht stabilisierende Komponente unter Lipiden, Lipidderivaten, Detergentien, Polyethylenglycolen, Proteinen, Peptiden, Polyamid-Oligomeren, pH-empfindlichen Polymeren und PEG-Lipiden ausgewählt ist.

12. Liposom nach Anspruch 11, wobei das PEG-Lipid ein PEG-Ceramid ist.

13. Liposom nach Anspruch 12, wobei das PEG ein durchschnittliches Molekulargewicht von 2000 bis 5000 Dalton hat.

14. Liposom nach einem der Ansprüche 1 bis 3, wobei der eingekapselte Komplex von Kondensationsmittel und Nukleinsäure eine mehr als etwa 30 %ige Einkapselungs-Wirksamkeit, bestimmt unter Verwendung von Picogreen und Dextransulfat, darstellt.

15. Verfahren zum Transfizieren einer Zelle mit einer Nukleinsäure in vitro, welches das Inkontaktbringen der Zelle mit einem Liposom nach einem der Ansprüche 1 bis 14 umfaßt.

**16.** Verfahren zum Einkapseln eines Komplexes von Kondensationsmittel und Nukleinsäure in einem Liposom, welches umfaßt:

die Zugabe einer Lösung eines Kondensationsmittels zu einer Nukleinsäure-Lösung, wobei ein Komplex aus Kondensationsmittel und Nukleinsäure gebildet wird, und
die Zugabe des Komplexes von Kondensationsmittel und Nukleinsäure zu einer Lipidsuspension unter Bildung des eingekapselten Komplexes von Kondensationsmittel und Nukleinsäure.

**17.** Verfahren nach Anspruch 16, wobei der Komplex aus Kondensationsmittel und Nukleinsäure gebildet wird, indem zuerst ein Kondensationsmittel zugemischt wird, wobei eine vorkondensierte Nukleinsäure gebildet wird, und danach die vorkondensierte Nukleinsäure zu einer zweiten Lösung eines Kondensationsmittels gegebenen wird, wobei der Komplex aus Kondensationsmittel und Nukleinsäure gebildet wird, und wobei das erste und das zweite Kondensationsmittel gleich oder verschieden sind.

**18.** Verwendung eines Liposoms nach einem der Ansprüche 1 bis 14 für die Herstellung eines Arzneimittels zum Transfizieren einer Zelle.


**Revendications**

**1.** Liposome comprenant :

(a) un lipide ; et
(b) un complexe agent de condensation/acide nucléique, encapsulé dans le liposome.

**2.** Liposome selon la revendication 1, dans lequel le lipide comprend un lipide non cationique.

**3.** Liposome selon la revendication 1 ou la revendication 2, dans lequel l'agent de condensation est choisi parmi les polyéthylène-imines, les polylysines, les polyarginines, les polyornithines, les histones, les protamines, les polyamines, la spermidine et la spermine.

**4.** Liposome selon la revendication 3, dans lequel l'agent de condensation est une polyéthylène-imine ayant une masse moléculaire de 0,8 à 800 kDa.

**5.** Liposome selon la revendication 4, dans lequel le rapport en poids polyéthylène-imine/acide nucléique dans le complexe agent de condensation/acide nucléique est de 10/1 à 1,5/1.

**6.** Liposome selon l'une quelconque des revendications 1 à 5, dans lequel le complexe agent de condensation/acide nucléique a un diamètre de 30 à 60 nm.

**7.** Liposome selon l'une quelconque des revendications 1 à 6, ayant un diamètre de 20 à 200 nm.

**8.** Liposome selon la revendication 7, ayant un diamètre de 50 à 150 nm.

**9.** Liposome selon la revendication 8, ayant un diamètre de 70 à 80 nm.

**10.** Liposome selon l'une quelconque des revendications 1 à 9, comprenant en outre :

(c) un composant stabilisant de bicouche associé à celui-ci.

**11.** Liposome selon la revendication 10, dans lequel le composant stabilisant de bicouche est choisi parmi les lipides, les dérivés de lipides, les détergents, les polyéthylèneglycols, les protéines, les peptides, les polyamides oligomères, les polymères sensibles au pH et les PEG-lipides.

**12.** Liposome selon la revendication 11, dans lequel le PEG-lipide est un PEG-céramide.

**13.** Liposome selon la revendication 12, dans lequel le PEG a une masse moléculaire moyenne de 2000 à 5000 daltons.

**14.** Liposome selon l'une quelconque des revendications 1 à 3, dans lequel le complexe agent de condensation/acide nucléique encapsulé représente une efficacité d'encapsulation supérieure à environ 30 %, telle que déterminée par utilisation de picogreen et de sulfate de dextrane.

**15.** Procédé pour transfecter une cellule avec un acide nucléique in vitro, le procédé comprenant la mise en contact de la cellule avec un liposome selon l'une quelconque des revendications 1 à 14.

**16.** Procédé pour encapsuler un complexe agent de condensation/acide nucléique dans un liposome, le procédé comprenant :

l'addition d'une solution d'agent de condensation dans une solution d'acide nucléique de façon à former ainsi un complexe agent de condensation/acide nucléique ; et
l'addition du complexe agent de condensation/acide nucléique à une suspension de lipide(s) de façon à former ainsi un complexe agent de condensation/acide nucléique, encapsulé.

**17.** Procédé selon la revendication 16, dans lequel le complexe agent de condensation/acide nucléique est formé par mélange d'un premier agent de condensation de façon à former ainsi un acide nucléique pré-condensé et ensuite addition de l'acide nucléique pré-condensé dans une solution d'un deuxième agent de condensation de façon à former ainsi le complexe agent de condensation/acide nucléique, et dans lequel les premier et deuxième agents de condensation sont identiques ou différents.

**18.** Utilisation d'un liposome selon l'une quelconque des revendications 1 à 14, pour la fabrication d'un médicament pour transfection d'une cellule.

# Fig. 1

*Fig. 2*

Encapsulated PEI Condensed DNA Liposomes

Legend:
- Picogreen
- Picogreen/Dextran sulfate
- Picogreen/Triton
- Picogreen/Dextran sulfate/Triton

EP 1 096 921 B1

*Fig. 3*

Titration of Dextran Sulfate to Determine Threshold
Amount Required to Unbind PEI Complexes

PEI/DNA 4:1 w/w
0.5 µg DNA
2.0 µg PEI

Dextran Sulfate Titration (µg)

Picogreen Fluorescence

Fig. 4

## *Fig. 5*

### Typical Time Release of DNA from PEI upon Addition of Dextran Sulfate

*Fig. 6*

## Fig. 7

### Titration of DOPS to Determine Optimal Encapsulation Efficiency

Fig. 8

Chi squared = 0.38

Relative Volume %

Size (nm)

Encapsulated PEI Condensed DNA Liposomes

## Fig. 9

Transfection of Cos-7 Cells with Encapsulated PEI Condensed
DNA Liposomes - Dose Response and Time Course

EP 1 096 921 B1

*Fig. 10*

Transfection of Various Tumor Cell Lines with
Encapsulated PEI Condensed DNA Liposomes

EP 1 096 921 B1

## Fig. 11

### *In Vitro* Toxicity of Encapsulated PEI Condensed DNA Liposomes in Cos-7 Cell Line

Encapsulated PEI Condensed DNA Liposomes

PEI Condensed DNA Complexes

EP 1 096 921 B1

Fig. 12

## Fig. 13

### In Vivo Gene Expression of PEI Condensed DNA Liposomes in Lewis Lung Tumor

Encapsulated PEI Condensed DNA Liposomes

EP 1 096 921 B1

## Fig. 14

### Gene Expression of Encapsulated PEI Condensed DNA in B16 i.p. Tumor (i.p. Injection)

A - Encapsulated PEI Condensed DNA Liposomes
Inex 351 - High Charge DNA Encapsulated Cationic Liposomes

## Fig. 15

In Vitro Toxicity Comparison of Pre- and Postpurification of
Encapsulated PEI Condensed DNA Liposomes

A - Prepurification
B - Postpurification

EP 1 096 921 B1